(19) Europäisches Patentamt
European Patent Office
Office européen des brevets

(11) **EP 2 438 902 B1**

(12) **EUROPEAN PATENT SPECIFICATION**

(45) Date of publication and mention
of the grant of the patent:
**26.02.2020 Bulletin 2020/09**

(21) Application number: **10783107.5**

(22) Date of filing: **26.05.2010**

(51) Int Cl.:
*A61K 8/31* *(2006.01)*          *A61K 8/34* *(2006.01)*
*A61K 8/37* *(2006.01)*          *A61K 8/81* *(2006.01)*
*A61K 8/86* *(2006.01)*          *A61Q 19/10* *(2006.01)*

(86) International application number:
**PCT/JP2010/003523**

(87) International publication number:
**WO 2010/140319 (09.12.2010 Gazette 2010/49)**

(54) **CLEANSING METHOD**

REINIGUNGSVERFAHREN

PROCÉDÉ DE NETTOYAGE DE LA PEAU

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **01.06.2009 JP 2009132439
01.06.2009 JP 2009132438**

(43) Date of publication of application:
**11.04.2012 Bulletin 2012/15**

(73) Proprietor: **Kao Corporation
Chuo-Ku
Tokyo 103-8210 (JP)**

(72) Inventor: **CHEN, Jenru
Tokyo 131-8501 (JP)**

(74) Representative: **Hoffmann Eitle
Patent- und Rechtsanwälte PartmbB
Arabellastraße 30
81925 München (DE)**

(56) References cited:
EP-A2- 1 433 476          WO-A1-99/12640
JP-A- 2002 241 260        JP-A- 2004 217 640
JP-A- 2004 339 212        JP-A- 2006 306 780
JP-A- 2008 184 413        JP-A- 2008 184 414
JP-A- 2008 184 415        JP-A- 2009 013 092
US-A- 3 277 013           US-A- 5 034 228
US-A1- 2007 025 947

• "johnsons baby oil / block pores ???", , 23
December 2005 (2005-12-23), Retrieved from the
Internet:
URL:https://www.healthboards.com/boards/ac
ne/351089-johnsons-baby-oil-block-pores.ht ml
[retrieved on 2018-07-06]

**Description**

TECHNICAL FIELD

The present invention relates to a method of cleansing skin.

BACKGROUND ART

Visible pores are one of the most bothersome problems of skin felt by women. This is largely ascribable to impurities formed in the pores (ranging from soft buttery impurities to hard solid impurities (keratotic plugs, for example)). These impurities in the pores are more difficult to remove as compared those on the surface of skin. Leaving the impurities in the pores unremoved, however, not only makes the pores more visible, but also induces various skin troubles including acne. It is therefore desirable to remove impurities from the pores, from the viewpoints of aesthetic and health of skin.
Methods of removing the hard solid impurities, in particular keratotic plugs, formed in the pores ever proposed include a method of placing a sheet like pack or spreading a face pack onto the skin, and then peeling the sheet like pack or the face pack off from the skin (see Patent Documents 1, 2).
On the other hand, as a method of removing keratotic plugs, there has been proposed a method of massaging skin, using a cosmetic which contains an oil component having a predetermined level of viscosity (see Patent Document 3).
US 2007/025947 relates to a cosmetic kit for treating human skin comprising a multi-unit receptacle containing: (a) at least one unit containing a skin preparatory composition containing at least one skin peeling agent; (b) at least one unit containing a keratolytic composition containing at least one anti-acne agent; (c) at least one unit containing a skin soothing composition; (d) optionally, at least one unit containing a maintenance composition having at least one agent chosen from a skin peeling agent and an anti-acne agent; (e) optionally, at least one unit containing a photoprotective composition.
EP 1433476 relates to a skin cleansing composition comprising (A) an oil component, (B) a hydrophilic nonionic surfactant, (C) a lipophilic amphiphile, (D) a water soluble solvent and (E) water. The composition has an isotropic liquid phase exhibiting a bicontinuous structure. The composition exhibits excellent detergency for the removal of both oil stains and water soluble stains and has high rinsability.
WO 99/12640 discloses a coated particle that comprises an internal core comprising a matrix and an exterior coating. The matrix consists essentially of at least one nanostructured liquid phase, or at least one nanostructured liquid crystalline phase or a combination of the two and the exterior coating comprising a nonlamellar crystalline material. Methods of making and methods of using the coated particle are disclosed.
US 5,034,228 relates to a composition comprising hydrous lipidic lamellar phases or liposomes containing, as an active agent a retinoid or a structural analogue of retinoid, such as a carotenoid or tretinoin. These compositions are more efficient against acne and less irritant for the skin and they are used as a pharmaceutical composition, notably dermatological or cosmetic.
JP 2009-013092 is directed to a two-part type cosmetic. The cosmetic contains a scrub agent as a first agent and a cosmetic composition containing 1 to 10 wt.% decaglycerol fatty acid ester eicosanoic diacid condensate, 80 to 99 wt.% polyhydric alcohol and 0.5 to 10 wt.% oil component, and having <=5 wt.% water content as a second agent. Further, as the first agent, an emulsified composition containing mannan as the scrub agent is used, and as the second agent, a carboxyvinyl polymer is added.
US 3,277,013 relates to a thixotropic skin cleaner, which is useful for cleansing the skin without requiring additional water for washing or rinsing. The skin cleaner is in the form of a viscous emulsion, which breaks and becomes thin fluid when subjected to mechanical stress upon use.

RELATED DOCUMENT

[PATENT DOCUMENT]

[Patent Document 1] Japanese Patent Publication No. JP-A-H09-194325
[Patent Document 2] Japanese Patent Publication No. JP-A-H08-109119
[Patent Document 3] Japanese Patent Publication No. JP-A-2002-241260

DISCLOSURE OF THE INVENTION

The conventional methods have, however, been suffering from the problems below.

According to Patent Document 1 and Patent Document 2, the sheet like pack is placed on the skin, or the face pack is spread so as to cover the pores, the sheet like pack or the face pack is allowed to absorb the keratotic plugs which are impurities in the pores, and the sheet like pack or the face pack is then peeled off, so as to concurrently remove the keratotic plugs. These methods were, however, inconvenient because the sheet like pack or the face pack, placed on the skin, need be dried before peeling-off.

On the other hand, the method described in Patent Document 3 was based on an effect of solubilizing the keratotic plugs exerted by an oil component contained in the base, and was effective in terms of removing the keratotic plugs to some extent, but was not yet fully satisfactory in terms of completely removing the keratotic plugs.

The present invention is to provide a method of cleansing skin, which is excellent in the effect of removing hard solid impurities formed in the pores, in particular keratotic plugs, and is labor-saving.

The present inventors have found that a method of cleansing skin, which is excellent in the effect of removing hard solid impurities formed in the pores, in particular keratotic plugs, and is labor-saving, may be achieved by:

(A) cleansing the skin using an agent which contains a water-miscible solvent, a surfactant and water, and has a continuous phase consisting of an aqueous phase; and then
(B) cleansing the skin using an agent which has a continuous phase consisting of an oil phase.

According to the present invention, there is provided a method of cleansing skin which includes:

(A) massaging the skin using an agent which contains a water-miscible solvent, a surfactant and water, the content of the water-miscible solvent being 10% by weight or more, and has a continuous phase consisting of an aqueous phase; and
(B) massaging the skin using an agent which has a continuous phase consisting of an oil phase,

wherein the process (A) precedes, and the process (B) succeeds immediately thereafter while leaving the agent of process (A) unremoved on the skin.

According to the present invention, there is also provided a method of cleansing skin which includes:

(A) massaging the skin using an agent which contains a water-miscible solvent, a surfactant and water, and has a continuous phase consisting of an aqueous phase;

(C) massaging the skin using an agent which contains an oil, a surfactant, a water-miscible solvent and water, and consists of an isotropic liquid phase in which each of an oil phase and an aqueous phase configures a continuous phase; and

(B) massaging the skin using an agent which has a continuous phase consisting of an oil phase,

wherein the process (A) precedes, the process (C) comes next, and the process (B) succeeds.

According to the present invention, there is still also provided a method of cleansing skin which includes:

(A) massaging the skin using an agent in the form of O/W-type emulsion, which has a continuous phase consisting of an aqueous phase;
(C) allowing water to vaporize off from the agent, so as to turn each of the oil phase and the aqueous phase into a continuous phase to thereby form the isotropic liquid phase, and massaging the skin using the thus-obtained agent consisting of an isotropic liquid phase; and
(B) allowing water to vaporize off from the agent consisting of the isotropic liquid phase, so as to turn the continuous phase into the oil phase, and massaging the skin using the thus-obtained agent which has a continuous phase consisting of an oil phase,

wherein the agent in the form of O/W-type emulsion contains components (j) to (n) below:

(j) not less than 5% by weight, and not more than 50% by weight of a nonionic surfactant which is configured by a single species of nonionic surfactant, or a mixed surfactant having two or more species of nonionic surfactants mixed therein, and has an HLB value of the single species of nonionic surfactant or the mixed surfactant of not less than 10, and not more than 15;
(k) not less than 10% by weight, and not more than 39% by weight of an oil having a viscosity at 30°C of not more than 15 mPa·s;
(l) not less than 10% by weight, and not more than 50% by weight of a compound which has 2 to 6 carbon atoms and one or two hydroxy group(s);

(m) not less than 0.01% by weight, and not more than 5% by weight of a polymer selected from water-soluble polymer which contains (meth)acrylic acid as a constituent, and acryloylmethyl taurate/vinyl pyrrolidone copolymer; and

(n) not less than 10% by weight, and not more than 50% by weight of water,

wherein a ratio by weight given by (j)/(k) is not less than 3/7, and not more than 3/1.

[Effect of the Invention]

According to the present invention, a method of cleansing skin, which is excellent in the effect of removing hard solid impurities formed in the pores, in particular keratotic plugs, and is labor-saving, may be provided.

BRIEF DESCRIPTION OF THE DRAWINGS

The above and other objects, features and advantages of the present invention will be more apparent from the following description of certain preferred embodiments taken in conjunction with the accompanying drawings listed below.

FIG. 1 is a phase diagram of a cleansing composition of a third embodiment;

FIG. 2 is a phase diagram of a cleansing composition of the third embodiment; and

FIG. 3 is a phase diagram of a cleansing composition of the third embodiment.

DESCRIPTION OF THE EMBODIMENTS

Three methods will be presented as the embodiments.

(First Embodiment)

The method of cleansing according to a first embodiment includes:

process (A): massaging the skin using an agent which contains a water-miscible solvent, a surfactant and water, and has a continuous phase consisting of an aqueous phase; and
process (B): massaging the skin using an agent which has a continuous phase consisting of an oil phase.
wherein the process (A) precedes, and the process (B) succeeds.

The first embodiment of the present invention relates to a method, according to which the agent having the continuous phase consisting of an aqueous phase is spread over the skin, and the skin is massaged (the process (A)); and then the agent having the continuous phase consisting of the oil phase is spread over the skin, and the skin is massaged (the process (B)). According to the method, an effect of dissolving hard solid impurities formed in the pores, in particular keratotic plugs, may be enhanced, and thereby an effect of removing keratotic plugs may be improved.

(Process (A))

The process (A) in the present invention is to massage the skin using the agent which contains a water-miscible solvent (component (a)), a surfactant (component (b)), and water (component (c)), and has the continuous phase consisting of an aqueous phase, so as to well mix the agent with impurities on the skin.

A compound which composes the component (a) used for the process (A) in the present invention is a water-miscible solvent, and is preferably any one of monohydric or dihydric alcohol having 2 to 6 carbon atoms, polyethylene glycols having 2 to 35 carbon atoms, and polypropylene glycols. From the viewpoint of strong cleansing power, specific examples enumerated herein include monohydric alcohols having 2-6 carbon atoms such as ethanol, propanol, isopropanol, butanol and isobutanol; glycols having 2-6 carbon atoms and having two hydroxy groups such as ethylene glycol, propylene glycol, diethylene glycol, dipropylene glycol, 1,3-butylene glycol, 1,4-butylene glycol, hexylene glycol, and isoprene glycol; and ethylene glycol alkyl ethers such as diethylene glycol monoethyl ether.

As the polyethylene glycols or polypropylene glycols, adoptable compounds include polyethylene glycols and polypropylene glycol having molecular weight of 1000 or smaller, and are exemplified by diethylene glycol, dipropylene glycol, diethylene glycol monoethyl ether, diethylene glycol monobutyl ether, and polyoxypropylene (9) diglyceryl. Among them, dipropylene glycol, diethylene glycol monoethyl ether, diethylene glycol monobutyl ether, and in particular, dipropylene glycol, and diethylene glycol monoethyl ether are preferable, by virtue of their desirable cleansing power exerted on oily impurities and water-soluble impurities.

Among these, from the viewpoint of accessibility to the pores, glycols having 2 to 6 carbon atoms are preferable, and in particular, glycols having 3 to 6 carbon atoms are preferable. For example, 1,3-butylene glycol, isoprene glycol, propylene glycol, and dipropylene glycol have excellent power of cleansing keratotic plugs.

As the component (a), a single species, or more species of compounds is used as much as not less than 10% by weight, preferably not less than 15% by weight, and more preferably not less than 18% by weight in the total composition from the viewpoint of cleansing power exerted on oily impurities and water-soluble impurities, whereas as much as not more than 50% by weight, and particularly not more than 40% by weight from viewpoint of good touch of use.

The component (b) is a surfactant, to which nonionic surfactant, anionic surfactant, and amphoteric surfactant are adoptable.

From the viewpoint of cleansing power, the component (b) is preferably a nonionic surfactant which may be configured by a single species of nonionic surfactant, or a mixed surfactant composed of two or more species of nonionic surfactants, having an HLB value of the single species of nonionic surfactant or the mixed surfactant of 10 to 18.

By adjusting the HLB of the mixed surfactant composing the component (b) to 10 to 18, the cleansing agent composition may be given as a solubilized system, or as an O/W-type emulsion.

The HLB (Hydrophilic-Lipophilic Balance) herein represents a ratio of molecular weight of the hydrophilic group portion relative to the total molecular weight of the surfactant, and is determined according to the Griffin's equation if the nonionic surfactant is a polyoxyethylene-based one.

The HLB of the mixed surfactant, configured by two or more species of nonionic surfactants, may be determined as described below.

For a system containing a plurality of nonionic surfactants mixed therein, the mixed-system HLB is obtained by calculating an arithmetical average of the HLB values of the individual nonionic surfactants based on their ratios of mixing.

$$\text{Mixed-system HLB} = \Sigma\ (\text{HLBx} \times \text{Wx}) / \Sigma \text{Wx}$$

HLBx represents an HLB value of nonionic surfactant X.
Wx represents weight (g) of the nonionic surfactant X having a value of HLBx.

The nonionic surfactant composing the component (b) used in the present invention is preferably configured by fatty acid ester having 8 to 22 carbon atoms, or ether of fatty alcohols having 8 to 22 carbon atoms typically from the viewpoint of cleansing power exerted on oily impurities, and those having hydroxy group, or ethylene oxide group as the hydrophilic functional group are preferable.

Specific examples include polyglycerin fatty acid ester, polyethylene glycol fatty acid ester, polyoxyethylene glycerin fatty acid ester, propylene glycol fatty acid ester, polyoxyethylene polyoxypropylene glycol, sorbitan fatty acid ester, polyoxyethylene sorbitan fatty acid ester, polyoxyethylene sorbit fatty acid ester, polyoxyethylene castor oil, polyoxyethylene hydrogenated castor oil, fatty acid ester of polyoxyethylene hydrogenated castor oil, polyalkyl glyceryl ether, polyoxyethylene alkyl ether, fatty acid ester of polyoxyethylene alkyl ether, sucrose fatty acid ester, alkyl polyglocoside, and (poly)alkyl glyceryl ether.

Among them, from the viewpoint of cleansing power, polyglycerin fatty acid ester, polyethylene glycol fatty acid ester, polyoxyethylene glycerin fatty acid ester, polyoxyethylene sorbit fatty acid ester, (poly)alkyl glyceryl ether, polyoxyethylene alkyl ether, sucrose fatty acid ester, and alkyl polyglucoside are excellent.

In particular, from an aspect of cleansing power exerted on oily impurities, diglycerin fatty acid ester, polyethylene glycol fatty acid ester, polyoxyethylene glycerin fatty acid ester, polyoxyethylene sorbit fatty acid ester, (poly)alkyl glyceryl ether, and alkyl polyglucoside are preferable.

Among these, from the viewpoint of excellence of cleansing power to be effected on keratotic plugs, and refreshing touch of use, preferable examples include diglycerin monooleate (HLB7), diglycerin monoisostearate (HLB8), polyoxyethylene (8) glyceryl monoisostearate (HLB9), polyoxyethylene sorbit tetraoleate (HLB11), polyoxyethylene (7) coconut oil fatty acid glycerin (HLB13), polyethylene glycol (12) monolaurate (HLB14), alkyl glucoside having 8 to 16 carbon atoms (HLB17), and 2-ethylhexyl glyceryl ether (HLB7), which may be used independently, or in a mixed form of two or more species.

As the components (b), two or more species of nonionic surfactants, having higher HLB and lower HLB, are preferably used in combination, for the purpose of further improving the stability.

More specifically, it is preferable to combine two or more species of nonionic surfactants so as to ensure a difference of 5 or larger between the highest HLB and the lowest HLB. In particular, a difference of 7 or larger will be successful in improving the stability of the cleansing agent composition.

From the viewpoint of cleansing power to be effected on oily impurities and water-soluble impurities, content of the component (b), in the total composition, is preferably not less than 1% by weight, and more preferably not less than 5% by weight. On the other hand, from the viewpoint of touch of use, the content of the component (b), in the total composition, is preferably not more than 50% by weight, more preferably not more than 40% by weight, and still more preferably not more than 32% by weight.

Water as the component (c) used in the present invention configures the balance, and is preferably contained as much as 20 to 70% by weight, preferably 30 to 60% by weight, and more preferably 38 to 56% by weight, of the total composition.

The cleansing agent may appropriately be added with generally adoptable components, such as oil, thickener, disinfectant, moisturizer, humectant, colorant, antiseptic, skin feel improver, perfume, antioxidant, and various liquid extracts.

By virtue of this configuration, a formulation having an aqueous phase as the continuous phase may have the aqueous phase in the outer phase. More specifically, solubilized system, O/W-type emulsion and so forth may be adoptable.

(Process (B))

The process (B) in the present invention is to massage the skin using the agent which has the continuous phase consisting of an oil phase, so as to mix the agent and impurities on the skin.

The agent having the continuous phase consisting of an oil phase preferably contains an oil as the component (d).

The oil composing the component (d) used in the process (B) in the present invention preferably stays in liquid at room temperature, and has a viscosity at 30°C of not more than 30 mPa·s. The viscosity herein is measured using a BM-type viscometer (from Tokimec Co., Ltd., measurement conditions: rotor No.1, 60 rpm, 30°C).

The low-viscosity oil of this sort is highly permeable into finely-profiled portions, has a strong power of solubilizing impurities, and thereby exhibits a strong cleansing power to be effected on hard solid impurities formed in the pores, in particular impurities such as keratotic plugs. From a particular viewpoint of moderating the oiliness and adjusting an appropriate level of touch of use, the component (d) having a viscosity at 30°C of not more than 10 mPa·s, and thereby having no heavy oiliness and ensuring good touch of use, is preferable.

Liquid oils generally adoptable to cosmetics may be adoptable to the oil. Specific examples include hydrocarbon oils such as liquid paraffin, liquid isoparaffin, hydrogenated polyisobutene, and squalane; ester oils such as cholesteryl isostearate, isopropyl palmitate, isopropyl myristate, neopentyl glycol dicaprylate, isopropyl isostearate, octadecyl myristate, cetyl 2-ethylhexanoate, isononyl isononanoate, isotridecyl isononanoate, glycerin tri(2-ethylhexanoate), and glycerin tri(caprylate/caprate); ether oils such as alkyl-1,3-dimethyl butyl ether, and nonyl phenyl ether; methylcyclopolysiloxanes such as decamethylcyclopentasiloxane, and octamethylcyclotetrasiloxane; silicone oils such as methylpolysiloxane, and methylphenylpolysiloxane; animal and botanical oils such as olive oil; and terpene oils.

Of these, oils having molecular weights of not more than 300 are preferable by virtue of their strong cleansing power. More specifically, hydrocarbon oils such as light liquid isoparaffin, and hydrogenated polyisobutene; ester oils such as isopropyl myristate, isopropyl palmitate, and isononyl isononanoate; and silicone oils such as octamethyl trisiloxane, and octamethyl cyclotetrasiloxane are exemplified. In particular, branched hydrocarbon oils having 8 to 18 carbon atoms are preferable, and isododecane is preferable.

The oil composing the component (d) may be configured by a mixture of hydrocarbon oil, ester oil, silicone oil and so forth. In this case, from the viewpoint of solubility to be effected on keratotic plugs, isoparaffin is preferably contained as much as not less than 30% of the component (d). From the viewpoint of odor, the component (d) had better not contain hydrocarbons having 8 to 9 carbon atoms.

The hydrocarbon oil is exemplified, by trade names, by Marukasol R (from Maruzen Petrochemical Co., Ltd.), IP Solvents 1620, 2028 (both from Idemitsu Kosan Co., Ltd.), Isopar L, Isopar H (both from Exxon Chemical Company), and Isosol 300, Isosol 400 (both from Shin-Nippon Petrochemical Co., Ltd.). Marukasol R is particularly preferable, by virtue of its high purity of isododecane.

From the viewpoint of cleansing power, the oil composing the component (d) is contained as much as not less than 50% by weight, and not more than 100% by weight, of the total composition. In particular, a content of not less than 70% by weight, and not more than 99% by weight is preferable. By the adjustment, quick and strong cleansing power may be ensured.

The cleansing agent may appropriately be added with generally-adoptable components such as nonionic surfactant, anionic surfactant, cationic surfactant, and amphoteric surfactant, typically for the purpose of adding readiness of rinsing, and also with water-miscible solvent, thickener, disinfectant, moisturizer, humectant, colorant, antiseptic, skin feel improver, perfume, antioxidant, various liquid extracts, and water.

A formulation having an oil phase as the continuous phase may have the oil phase in the outer phase. More specifically, oil system, W/O-type emulsion, mixture of oil and surfactant, and so forth may be adoptable.

According to the first embodiment of the present invention, by massaging first the skin using the agent having an aqueous phase as the continuous phase (the process (A)), impurities at around the pores and metabolites yielded from the skin may be swelled, and thereby the surficial impurities are made more readily be removed. Next, by massaging the skin using the agent having an oil phase as the continuous phase (the process (B)), the more hard solid sebum in the pores may be dissolved by the component (d). In this way, an excellent effect of dissolving hard solid impurities formed in the pores, in particular keratotic plugs, low stimulation to the skin, and improved effect of removing keratotic plugs are ensured.

According to the present invention, the process (B) succeeds the process (A) immediately thereafter, while leaving the agent in the process (A) unremoved on the skin. In this way, the impurities in the pores may thoroughly be removed.

Now, technical levels achievable by the conventional techniques will be explained. According to the conventional techniques for cleansing the skin, first, an oil is applied to the skin, so as to mix the oil with makeup materials or the like. The skin is then rinsed with water to remove the oil, and the skin is again cleansed using an agent having the continuous phase composed of an aqueous phase.

Since oil-containing impurities such as makeup materials are relatively less compatible with the cleansing agent having the continuous phase composed of an aqueous phase, so that it has generally been understood that preliminary lift-up and cleansing of the makeup materials using an oil is effective. While the technique is capable of removing the makeup materials and so forth, it is still difficult to remove the impurities in the pores.

In contrast, the present inventors have found that sebum in the pores may be dissolved and the hard solid impurities formed in the pores, in particular keratotic plugs, may thoroughly be removed, by cleansing the skin by the procedures reverse to those of the conventional method of cleansing, that is, by preliminarily cleansing the skin using the agent having the continuous phase consisting of an aqueous phase, and then by cleansing the skin using the agent having the continuous phase consisting of an oil phase. By preliminarily using the agent having the continuous phase consisting of an aqueous phase, the surficial impurities which reside at around the pores are supposed to become more readily removable, making the cleansing agent, which has the continuous phase consisting of an oil phase, more deeply accessible into the pores, and also making the impurities deep inside the pores thoroughly removable.

In addition in this embodiment, since the skin is massaged using the agent (cleansing liquid), the process is less stimulative as compared with the conventional methods of using sheet like pack or face pack, and can save labor for drying the sheet like pack or face pack.

The process (B) is followed by the process (D) of applying water to remove the agent from the skin. As a consequence, also the cleansing agent is removed from the skin together with the keratotic plugs. The agents used in the process (A) and process (B) are removed from the skin, without being impregnated into the skin.

(Second Embodiment)

Next, a second embodiment of the present invention will be explained.

The method of cleansing skin of this embodiment includes:

(A) massaging the skin using an agent which contains a water-miscible solvent, a surfactant and water, and has a continuous phase consisting of an aqueous phase;
(C) massaging the skin using an agent which contains an oil, a surfactant, a water-miscible solvent and water, and consists of an isotropic liquid phase in which each of an oil phase and an aqueous phase configures a continuous phase; and
(B) massaging the skin using an agent which has a continuous phase consisting of an oil phase,

wherein the process (A) precedes, the process (C) comes next, and the process (B) succeeds.

The second embodiment of the present invention relates to a method, according to which the skin is massaged using the agent having the continuous phase consisting of an aqueous phase; the skins is then massaged using the agent consisting of the isotropic liquid phase; and the skin is massaged using the agent having the continuous phase consisting of an oil phase. According to the method, an effect of dissolving hard solid impurities formed in the pores, in particular keratotic plugs, may be enhanced, and thereby an effect of removing keratotic plugs may be improved.

The agents (cleansing liquids) used in the process (A) and the process (B) may be same as those used in the first embodiment.

The agent (cleansing liquid) used in the process (C) of the present invention contains an oil, a surfactant, a water-miscible solvent, and water, and consists of an isotropic liquid phase. While the isotropic liquid phase herein generally includes micellar solution, the isotropic liquid phase in the context herein means a state in which either of the aqueous phase and the oil phase, or both of which are solubilized. The state in which each of the aqueous phase and the oil phase forms the continuous phase, to thereby give an optically-isotropic transparent or translucent liquid phase, is referred to as bicontinuous phase. The agent (cleansing liquid) consists of the isotropic liquid phase having both phases dissolved therein exhibits a large wettability, quickly accesses to hard solid impurities formed in the pores, in particular keratotic plugs, and can enhance an effect of dissolving the keratotic plugs.

For example, the cleansing agent compositions disclosed in Japanese Patent Publication Nos. JP-A-2004-217640, JP-A-2008-184413, and JP-A-2008-184414 may be adoptable.

For example, a cleansing agent composition which contains (e) 3 to 80% by weight of an oil, (f) 1 to 45% by weight of a hydrophilic nonionic surfactant, (g) 1 to 45% by weight of a lipophilic/amphiphilic substance, (h) 3 to 80% by weight of a water-miscible solvent, and (i) 3 to 80% by weight of water, and is configured by a bicontinuous phase may be adoptable.

Liquid oils may be adoptable to the oil composing the component (e), and examples thereof include hydrocarbon oils such as liquid paraffin, liquid isoparaffin, and squalane; ester oils such as cholesteryl isostearate, isopropyl palmitate, isopropyl myristate, neopentylglycol dicaprate, isopropyl isostearate, octadecyl myristate, cetyl 2-ethylhexanoate, isononyl isononanoate, isotridecyl isononanoate, glycerin tri(2-ethylhexanoate), and glycerin tri(caprylate/caprate); ether oils such as alkyl-1,3-dimethyl butyl ether, and nonyl phenyl ether; methylcyclopolysiloxanes such as decamethylcyclopentasiloxane, and octamethylcyclotetrasiloxane; silicone oils such as methylpolysiloxane, and methylphenylpolysiloxane; animal and botanical oils such as olive oil; and terpene oils.

In addition, those having values of viscosity at 25°C of 30 mPa·s or smaller are preferable. The viscosity herein is measured using a BM-type viscometer (from Tokimec Co., Ltd., measurement conditions: rotor No.1, 60 rpm).

Of these, liquid paraffin, liquid isoparaffin, neopentyl glycol dicaprate, isopropyl isostearate, cetyl 2-ethylhexanoate, isononyl isononanoate, glycerin tri(caprylate/caprate), alkyl-1,3-dimethyl butyl ether, decamethylcyclopentasiloxane, and octamethylcyclotetrasiloxane are preferable, in particular, liquid isoparaffin, glycerin tri(caprylate/caprate), alkyl-1,3-dimethyl butyl ether, decamethylcyclopentasiloxane are preferable, and liquid isoparaffin is more preferable. As the liquid isoparaffin, in particular, hydrogenated polyisobutene is preferable, wherein a species having a degree of polymerization of isobutene of 3 to 6 is preferable, from the viewpoint of cleansing power to be effected on oily impurities.

The component (e) may be configured by two or more species, and the content of which in the total composition is 3 to 80% by weight, preferably 5 to 65% by weight, more preferably 7 to 56% by weight, and still more preferably 14 to 16% by weight. By adjusting the content to not less than 3% by weight, a sufficient level of cleansing power to be effected on oily impurities may be ensured, while ensuring also a satisfactory level of readiness of rinsing. By adjusting the content to not more than 80% by weight, a desirable level of cleansing power to be effected on water-soluble impurities may be ensured.

The hydrophilic nonionic surfactant composing the component (f) preferably has an HLB value exceeding 8, and particularly preferably has an HLB value of 9 to 20. More specifically, an HLB value of 11 to 17 is preferable. The HLB herein represents a ratio of molecular weight of the hydrophilic group portion relative to the total molecular weight of the surfactant, and is determined according to the Griffin's equation if the nonionic surfactant is a polyoxyethylene-based one.

More specifically, specific examples include polyethylene glycol fatty acid ester such as polyethylene glycol (12) monolaurate; polyethylene glycol alkyl ether such as polyethylene glycol (20) octyl dodecyl ether; polyethylene glycol alkylphenyl ether such as polyethylene glycol (20) nonylphenyl ether; polyethylene glycol castor oil derivative such as polyethylene glycol (50) castor oil; polyethylene glycol hydrogenated castor oil derivative such as polyethylene glycol (60) hydrogenated castor oil monoisolaurate; polyethylene glycol-based surfactant such as polyethylene glycol (20) sorbitan monostearate; polyglycerin fatty acid ester such as monooleic acid diglycerin; polyoxyethylene glycerin fatty acid ester such as polyoxyethylene (8) glyceryl monoisostearate; polyglycerin alkyl ether such as diglycerin 2-ethyl hexyl ether; sucrose fatty acid ester such as sucrose stearate; and alkyl glucoside. Of these, those having $C_8$ or longer, and particularly $C_{12}$ or longer hydrophobic group are preferable, by virtue or their readiness of rinsing.

The component (f) may be used independently, or in a mixed form of two or more species thereof, as much as 1 to 45% by weight of the total composition, preferably 1 to 40% by weight, and more preferably 9 to 24% by weight. A desirable level of the readiness of rinsing may be ensured by adjusting the content to not less than 1% by weight, and a desirable level of cleansing power may be ensured by adjusting the content to not more than 45% by weight.

The lipophilic/amphiphilic substance composing the component (g) is preferably a nonionic surfactant having an HLB value of not more than 8, fatty alcohol having 8 to 25 carbon atoms, fatty acid having 8 to 25 carbon atoms, or monoalkyl phosphate having a alkyl group having 8 to 25 carbon atoms. They preferably have a hydrophobic group having not less than 8 carbon atoms and particularly not less than 12 carbon atoms in view of achieving strong cleansing power.

The nonionic surfactants having an HLB value of not more than 8 are exemplified by ethylene glycol fatty acid esters such as ethylene glycol monostearate; polyethylene glycol-based surfactants which include polyethylene glycol fatty acid esters such as polyethylene glycol (2) monostearate, polyethylene glycol alkyl ethers such as polyethylene glycol (5) decyl pentadecyl ether, and polyethylene glycol hydrogenated castor oil derivatives such as polyethylene glycol (5) hydrogenated castor oil monoisolaurate; propylene glycol-based surfactants such as propylene glycol fatty acid ester, polypropylene glycol fatty acid ester, propylene glycol alkyl ether, polypropylene glycol alkyl ether, and oxyethylene derivative of propylene glycol alkyl ether; glycerin fatty acid esters such as glycerin monoisostearate; glycerin alkyl ethers such as glycerin monoisostearyl ether; sorbitan fatty acid esters such as sorbitan monostearate; and fatty acid dialkanolamides such as fatty acid alkanolamide, and lauric acid diethanolamide. Of these, those having HLB values of 6 or smaller are preferable, by virtue of their strong cleansing power to be effected on oily impurities and water-soluble impurities.

The fatty alcohol may be any monohydric or polyhydric alcohol having straight-chain or branched, saturated or unsaturated hydrocarbon group having 8 to 25 carbon atoms, preferably 12 to 22 carbon atoms, and is exemplified by octanol, lauryl alcohol, myristyl alcohol, isomyristyl alcohol, palmityl alcohol, isopalmityl alcohol, stearyl alcohol, isostearyl alcohol, behenyl alcohol, oleyl alcohol, linoleyl alcohol, and linolenyl alcohol. Of these, lauryl alcohol, myristyl alcohol, isomyristyl alcohol, isopalmityl alcohol, isostearyl alcohol, and oleyl alcohol are preferable, and in particular, lauryl alcohol, myristyl alcohol, and isostearyl alcohol are preferable, by virtue of their strong cleansing power to be effected on oily impurities and water-soluble impurities.

The fatty acid may be any straight-chain or branched, saturated or unsaturated one having 8 to 25 carbon atoms, preferably 12 to 22 carbon atoms, and is exemplified by lauric acid, myristic acid, isomyristic acid, palmitic acid, isopalmitic acid, stearic acid, behenic acid, isostearic acid, oleic acid, linolic acid, and linolenic acid. Of these, lauric acid, myristic acid, isomyristic acid, isopalmitic acid, isostearic acid, oleic acid, linolic acid, and linolenic acid are preferable, and in particular, lauric acid, myristic acid, and isostearic acid are preferable, by virtue of their strong cleansing power to be effected on oily impurities and water-soluble impurities.

The monoalkyl phosphate may have a straight-chain or branched alkyl group having 8 to 25 carbon atoms, preferably 12 to 22 carbon atoms, and is exemplified by monolauryl phosphate, monomyristyl phosphate, monopalmityl phosphate, monostearyl phosphate, monobehenyl phosphate, monoisostearyl phosphate, and mono-2-hexyldecyl phosphate. Of these, monolauryl phosphate, monomyristyl phosphate, and mono-2-hexyldecyl phosphate are preferable, and in particular, monolauryl phosphate, and monomyristyl phosphate are preferable, by virtue of their strong cleansing power.

The component (g) may be used also in a mixed form of two or more species thereof, as much as 1 to 45% by weight of the total composition, and preferably 1 to 40% by weight. Desirable levels of cleansing power and readiness of rinsing may be ensured by adjusting the content to not less than 1% by weight, and desirable levels of cleansing power and readiness of rinsing may be ensured by adjusting the content to not more than 45% by weight.

The hydrophilic nonionic surfactant composing the component (f), and the lipophilic/amphiphilic substance composing the component (g), are preferably used in the present invention so as to adjust ratio by weight (f)/(g) of 0.5 to 8, in view of achieving readiness of rinsing and strong cleansing power.

The water-miscible solvent composing the component (h) used in the present invention is preferably any of monohydric or polyhydric alcohols having 1 to 6 carbon atom(s), polyethylene glycols, polypropylene glycols, saccharide, and water-soluble fatty acids.

The component (h) is preferably a species capable of enhancing hydrophilicity of the hydrophilic nonionic surfactant composing the component (f), and the lipophilic/amphiphilic substance composing the component (g). The property of enhancing hydrophilicity of the hydrophilic nonionic surfactant composing the component (f), and the lipophilic/amphiphilic substance composing the component (g), typically means that the clouding point (cloud point) of the nonionic surfactant may be elevated by adding the component (h), which is described, for example, by Sagitani et al., Yukagaku, 33(3), p.156-161 (1984).

The monohydric alcohols having 1 to 6 carbon atom(s) are exemplified by ethanol, propanol, isopropanol, butanol, and isobutanol, and the polyhydric alcohol is exemplified by ethylene glycol, propylene glycol, isoprene glycol, 1,3-butylene glycol, hexylene glycol, trimethylolpropane, glycerin, and sorbit. Of these, the monohydric alcohol is preferably ethanol, propanol or isopropanol, and particularly ethanol; and the polyhydric alcohol is preferably propylene glycol, isoprene glycol, 1,3-butylene glycol, or hexylene glycol, and particularly, isoprene glycol or hexylene glycol, by virtue of their desirable levels of cleansing power to be effected on oily impurities and water-soluble impurities.

As the polyethylene glycols or the polypropylene glycols, polyethylene glycols having molecular weights of not more than 1000, and polypropylene glycols having molecular weights of not more than 200 may be adoptable, wherein examples of which include diethylene glycol, dipropylene glycol, diethylene glycol monoethyl ether, and diethylene glycol monobutyl ether. Of these, dipropylene glycol, diethylene glycol monoethyl ether, and diethylene glycol monobutyl ether are preferable, and particularly, dipropylene glycol, and diethylene glycol monoethyl ether are preferable, by virtue of their desirable levels of cleansing power to be effected on oily impurities and water-soluble impurities.

The saccharides are exemplified by erythritol, pentaerythritol, methyl glucoside, ethyl glucoside, polyoxyethylene methyl glucoside, and polyoxypropylene methyl glucoside, wherein alkyl glucoside having a $C_2$ or shorter alkyl chain is preferable. Of these, methyl glucoside, ethyl glucoside, polyoxyethylene methyl glucoside, and polyoxypropylene methyl glucoside are preferable, and particularly, polyoxyethylene methyl glucoside, and polyoxypropylene methyl glucoside are preferable, by virtue of their desirable levels of cleansing power to be effected on oily impurities and water-soluble impurities.

The water-soluble fatty acids are ones having 1 to 6 carbon atom(s), and are exemplified by acetic acid, propionic acid, and butanoic acid, wherein acetic acid and propionic acid are preferable, and in particular propionic acid is preferable, by virtue of their desirable levels of cleansing power to be effected on oily impurities and water-soluble impurities.

The component (h) may be used also in a mixed form of two or more species thereof, as much as 3 to 80% by weight of the total composition, preferably 5 to 70% by weight, and more preferably 10 to 50% by weight. A desirable level of cleansing power to be effected on oily impurities may be ensured by adjusting the content to not less than 3% by weight, and a desirable level of cleansing power to be effected on oily impurities may be ensured by adjusting the content to not more than 80% by weight.

Ratio of weight of the component (h), relative to the total of the hydrophilic nonionic surfactant composing the component (f) and the lipophilic/amphiphilic substance composing the component (g), namely (h)/((f)+(g)), is preferably adjusted to not less than 1, in view of achieving a desirable level of cleansing power.

Water as the component (i) configures the balance, and is preferably contained as much as 3 to 80% by weight, preferably 5 to 75% by weight, and more preferably 10 to 65% by weight, of the total composition. A desirable level of cleansing power to be effected on water-soluble impurities may be ensured by adjusting the content to not less than 3% by weight, and a desirable level of cleansing power to be effect on oily impurities may be ensured by adjusting the content to not more than 80% by weight.

The agent composed of an isotropic liquid phase is not limited to those described in the above.

Although the process (C) may succeed the process (A) while placing rinsing of skin with water in between, it is more preferable that the process (C) succeeds the process (A) immediately thereafter, while leaving the agent in the process (A) unremoved on the skin. Although the process (B) may succeed the process (C) while placing rinsing of skin with water in between, it is more preferable that the process (B) succeeds the process (C) immediately thereafter, while leaving the agent in the process (C) unremoved on the skin. In this way, the impurities in the pores may thoroughly be removed.

In the second embodiment, first in the process (A), the skin is massaged using the cleansing agent which has the continuous phase composed of an aqueous phase.

The cleansing agent having the continuous phase composed of an aqueous phase may swell impurities at around the pores and metabolites yielded from the skin, and thereby removes the surficial impurities.

Next, in the process (C), the skin is massaged using the agent which is composed of an isotropic liquid phase in which each of an oil phase and an aqueous phase configures a continuous phase.

The agent exhibits a large wettability, good accessibility to the skin, and is permeable into finely-profiled portions. Accordingly, the agent permeates through portions where the surficial impurities were removed, and accesses more deeply into the pores.

Next, in the process (B), the skin is massaged using the agent which has a continuous phase composed of an oil phase.

By using the cleansing agent, more solidified sebum deep inside the pores may be dissolved with the aid of the component (d). The agent is excellent in the effect of dissolving hard solid impurities formed in the pores, in particular keratotic plugs, less stimulative to skin, and is enhanced in the effect of removing keratotic plugs.

The process (B) is followed by the process (D) of applying water to remove the agent from the skin. As a consequence, also the cleansing agent is removed from the skin together with the keratotic plugs. The agents used in the process (A), the agent used in the process (C), and the agent used in the process (B) are removed from the skin, without being impregnated into the skin. Since this embodiment adopts the individual processes of massaging the skin with the agents (cleansing liquid), so that the embodiment no longer needs drying of the agent and is more convenient, as compared with the conventional methods of using sheet or face pack.

Intervals of the adjacent processes (for example, length of time after completion of the process (A) up to start of the process (C)) preferably falls in a period after last behavior in the preceding process and before the skin dries. More specifically, after the preceding process, the next process preferably starts within one hour, more preferably within 30 minutes, still preferably within 10 minutes, and preferably starts immediately after the preceding process.

(Third Embodiment)

The method of cleansing skin of this embodiment includes:

(A) massaging the skin using an agent in the form of O/W-type emulsion, which contains a nonionic surfactant (component (j)), an oil (component (k)), a compound having carbon atoms 2 to 6 and having one or two hydroxy group(s) (component (l)), a specific water-soluble polymer (component (m)) and water (component (n)), and has a continuous phase consisting of an aqueous phase;

(C) massaging the skin using an agent consisting of an isotropic liquid phase in which each of the oil phase and the aqueous phase configures a continuous phase, obtained by allowing water to vaporize off from the agent, so as to turn the continuous phase into the isotropic liquid phase; and

(B) massaging the skin using an agent which has a continuous phase consisting of an oil phase, obtained by allowing water to vaporize off from the agent consisting of the isotropic liquid phase, so as to turn the continuous phase into the oil phase.

This embodiment will be explained below.

In this embodiment, the component (j) is a nonionic surfactant, composed of a single species of nonionic surfactant, or a mixed surfactant composed of two or more species of nonionic surfactants, having an HLB value of the single species of nonionic surfactant or the mixed surfactant of not less than 10, and not more than 15.

By adjusting the HLB of the mixed surfactant composing the component (j) to not less than 10 and not more than 15, the cleansing agent composition may be given as an O/W-type emulsion.

The HLB (hydrophilic-lipophilic balance) herein may be calculated according to the method described in the first embodiment.

The nonionic surfactants for composing the component (j) used for the present invention are not specifically limited so long as they satisfy the above-described conditions, typically such as those composed of fatty acid ester having 8 to 22 carbon atoms or, ether of fatty alcohols having 8 to 22 carbon atoms, and having hydroxy group and ethylene oxide group as the hydrophilic functional groups.

More specifically, polyglycerin fatty acid ester, polyethylene glycol fatty acid ester, polyoxyethylene glycerin fatty acid ester, propylene glycol fatty acid ester, polyoxyethylene polyoxypropylene glycol, sorbitan fatty acid ester, polyoxyethylene sorbitan fatty acid ester, polyoxyethylene sorbit fatty acid ester, polyoxyethylene castor oil, polyoxyethylen hydrogenated castor oil, polyoxyethylene-hydrogenated castor oil fatty acid ester, polyalkyl glyceryl ether, polyoxyethylene alkyl ether, polyoxyethylene alkyl ether fatty acid ester, sucrose fatty acid ester, alkyl polyglucoside, and (poly)alkyl glyceryl ether, are exemplified.

Among them, from the viewpoint of cleansing power, polyglycerin fatty acid ester, polyethylene glycol fatty acid ester, polyoxyethylene glycerin fatty acid ester, polyoxyethylene sorbit fatty acid ester, (poly)alkyl glyceryl ether, polyoxyethylene alkyl ether, sucrose fatty acid ester, and alkyl polyglucoside are excellent.

In particular, from the viewpoint of cleansing power to be effected on oily impurities, diglycerin fatty acid ester, polyethylene glycol fatty acid ester, polyoxyethylene glycerin fatty acid ester, polyoxyethylene sorbit fatty acid ester, (poly)alkyl glyceryl ether, and alkyl polyglucoside are preferable.

Among them, from the viewpoints of contribution to building up of the isotropic liquid phase formed by the components (j) to (n), excellence of the cleansing power to be effected on keratotic plugs, and refreshing touch of use, diglycerin monoisostearate (HLB8), polyoxyethylene (8) glyceryl monoisostearate (HLB9), polyoxyethylene sorbit tetraoleate (HLB11), polyoxyethylene (7) coconut oil fatty acid glycerin (HLB13), polyethylene glycol (12) monolaurate (HLB14), alkyl glucoside having 8-16 carbon atoms (HLB17), 2-ethylhexyl glyceryl ether (HLB7), diglycerin monooleate, and isostearyl glyceryl ether are preferable, all of which may be used independently, or in a mixed form of two or more species.

As the components (j), two or more species of nonionic surfactants, having higher HLB and lower HLB, are preferably used in combination, for the purpose of further improving the stability.

More specifically, it is preferable to combine two or more species of nonionic surfactants so as to ensure a difference of 5 or larger between the highest HLB and the lowest HLB. In particular, a difference of 7 or larger will be successful in improving the stability of the cleansing agent composition.

Content of the component (j) is adjusted to not less than 5% by weight, and not more than 50% by weight of the total composition. The adjustment enables formation of a specific phase state described later.

Content of the component (j) is adjusted to not less than 5% by weight, particularly not less than 10% by weight, and preferably not less than 16% by weight. By the adjustment, a good accessibility to the skin may be ensured, and impurities in the pores, including keratotic plugs, may be lifted up. On the other hand, the content of the component (j) is not more than 50% by weight of the total composition. In particular, the content of the component (j) is preferably adjusted to not more than 40% by weight, and particularly to not more than 33% by weight. By the adjustment, the cosmetic may thoroughly be rinsed off, without persistent feel of retention.

(Component (k))

The oil composing the component (k) of the present invention stays in liquid at room temperature, and has a viscosity at 30°C of not more than 15 mPa·s. The viscosity herein is measured using a BM-type viscometer (from Tokimec Co., Ltd., measurement conditions: rotor No.1, 60 rpm, 30°C).

The low-viscosity oil of this sort is well accessible to keratotic plugs formed in the pores or therearound, and has a strong power of solubilizing impurities, and thereby exhibits a strong cleansing power to be effected on hard solid impurities formed in the pores, in particular impurities such as keratotic plugs. In addition, it has no heavy oiliness, and ensures good touch of use.

From the viewpoints of moderating the oiliness and adjusting an appropriate level of touch of use, the component (k) having a viscosity at 30°C of not more than 10 mPa·s is preferable.

Among liquid oils generally adoptable to cosmetics, those satisfying the above-described conditions are adoptable herein. Specific examples include hydrocarbon oils such as liquid paraffin, liquid isoparaffin, hydrogenated polyisobutene, squalane, and isododecane; ester oils such as cholesteryl isostearate, isopropyl palmitate, isopropyl myristate, neopentyl glycol dicaprate, isopropyl isostearate, octadecyl myristate, cetyl 2-ethylhexanoate, isononyl isononanoate, isotridecyl isononanoate, glycerin tri(2-ethylhexanoate), and glycerin tri(caprylate/caprate); ether oils such as alkyl-1,3-dimethyl butyl ether, and nonyl phenyl ether; methyl cyclopolysiloxanes such as decamethyl cyclopentasiloxane, and octamethyl cyclotetrasiloxane; silicone oils such as methyl polysiloxane, and methyl phenyl polysiloxane; animal and botanical oils such as olive oil; and terpene oils.

Among them, oils having molecular weights of not more than 300 are preferable by virtue of their strong cleansing power. Specific examples include hydrocarbon oils such as light liquid isoparaffin, and hydrogenated polyisobutene; ester oils such as isopropyl myristate, isopropyl palmitate, and isononyl isononanoate; and silicone oils such as octamethyl trisiloxane, and octamethyl cyclotetrasiloxane. In particular, branched hydrocarbon oils having carbon atoms 8 to 18 are preferable, and isododecane is preferable.

The oil composing the component (k) may be configured by a mixture of hydrocarbon oil, ester oil, silicone oil and so forth. In this case, isoparaffin is preferably contained as much as not less than 30% by weight of the component (k), and preferably not less than 42% by weight. From the viewpoint of odor, the component (k) had better not contain hydrocarbons having carbon atoms 8 to 9.

The hydrocarbon oil is exemplified by trade names by Marukasol R (from Maruzen Petrochemical Co., Ltd.), IP Solvents 1620, 2028 (both from Idemitsu Kosan Co., Ltd.), Isopar L, Isopar H (both from Exxon Chemical Company), and Isosol 300, Isosol 400 (both from Shin-Nippon Petrochemical Co., Ltd.). Marukasol R is particularly preferable, by virtue of its high purity of isododecane.

The oil composing the component (k) is contained as much as not less than 8% by weight, particularly not less than 10% by weight, and not more than 39% by weight, particularly not more than 30% by weight of the total composition. In particular, the content is preferably adjusted to not less than 15% by weight, and not more than 24% by weight. By the adjustment, the cosmetic may thoroughly be rinsed off without persistent feel of retention, while ensuring a sufficient level of cleansing power.

The compound composing the component (l) used in the present invention is the compound which has carbon atoms 2 to 6 and one or two hydroxy group(s). This contributes to building up of the O/W-type emulsion composed of the components (j) to (n), and the isotropic liquid phase. From the viewpoint of strong cleansing power, specific examples include monohydric alcohols having carbon atoms 2-6 such as ethanol, propanol, isopropanol, butanol, and isobutanol; glycols having carbon atoms 2 to 6 such as ethylene glycol, propylene glycol, diethylene glycol, dipropylene glycol, 1,3-butylene glycol, 1,4-butylene glycol, hexylene glycol, and isoprene glycol; and ethylene glycol alkyl ethers such as diethylene glycol monoethyl ether.

Among them, glycols, for example, 1,3-butylene glycol, isoprene glycol, propylene glycol, and dipropylene glycol are excellent in terms of building up of the isotropic liquid phase which is stabilized over a wide range.

As the component (l), one or more species of compound(s) may be used, as much as not less than 10% by weight of the total composition, from the viewpoint of achieving a sufficient level of cleansing power. It is preferable to adjust the content to not more than 50% by weight of the total composition, particularly not more than 40% by weight, more particularly not more than 30% by weight, and still more particularly not more than 20% by weight, from the viewpoint of good feel of use. Moreover, the content of the component (l) adjusted to not less than 10% by weight, and not more than 50% by weight, enables formation of a specific phase state described later.

To the component (m) used in the present invention, species selected from water-soluble polymers which contain constituents derived from (meth)acrylic acid, and acryloylmethyl taurate/vinyl pyrrolidone copolymers may be used singly, or in combination of two or more species.

The water-soluble polymers which contain constituents derived from (meth)acrylic acid are exemplified by acrylate/ alkyl methacrylate copolymers, and more specifically crosslinked copolymers formed by acrylic acid and (C10-30) alkyl methacrylate, which is commercially available typically under the trade names of PEMULEN TR-1, PEMULEN TR-2, and Carbopol ETD2020 (from Lubrizol Advanced Materials, Inc.)

The water-soluble polymers, composing the component (m), which contain the constituents derived from (meth)acrylic acid, preferably have all of, or a part of, the (meth)acrylic acid units thereof neutralized with an alkali agent. The alkali agent used for neutralization is not specifically limited so long as it is generally miscible into cosmetics, and is exemplified by potassium hydroxide and sodium hydroxide. The alkali agent may be used singly, or in combination of two or more species, as much as not less than 0.01% by weight, and not more than 5% by weight of the total composition, so as to preferably adjust pH of the system to 5.5 to 9, particularly 6 to 8.

The acryloylmethyl taurate/vinyl pyrrolidone copolymer composing the component (m) is exemplified by acryloyldimethyl taurine ammonium/VP copolymer which is commercially available under the trade name of Aristoflex AVC (from Clariant), the content of which is preferably adjusted to not less than 0.01% by weight, and not more than 5% by weight of the total composition, so as to adjust the pH of the system to 4 to 8.5, particularly 5 to 7.

The water-soluble polymers which contains (meth)acrylic acid as a constitutive monomer, or acryloylmethyl taurate/vinylpyrrolidone copolymer, composing the component (m), may be used singly, or in combination of two or more species.

As the component (m), acrylate/ alkyl methacrylate copolymer is particularly preferable, from the viewpoint of forming an O/W-type emulsified composition.

In the isotropic liquid phase formed by the components (j), (k), (l) and (n), the component (m) added thereto contributes to form an O/W-type emulsified area having the continuous phase thereof composed of an aqueous phase. The cleansing agent given in a formulation of O/W-type emulsion takes advantages in swelling impurities around the pores and metabolites yielded from the skin, thereby making the surficial impurities more readily removable, and enhancing the effect of removing keratotic plugs.

Content of the component (m) is preferably not less than 0.01% by weight, and not more than 5% by weight of the total composition. The content adjusted to not less than 0.2% by weight, and not more than 4% by weight, and further adjusted to not more than 3% by weight, is preferable, in view of achieving an enhanced effect of removing keratotic plugs, and building-up of a stable O/W-type emulsion, and an isotropic liquid phase.

Water composing the component (n) used in the present invention is contained as much as not less than 10% by weight, and not more than 50% by weight of the total composition. The content is particularly preferably adjusted to not less than 20% by weight, and not more than 40% by weight. By the adjustment, water contributes to build up the O/W-type emulsion and the isotropic liquid phase, and thereby the cosmetic may readily be rinsed off without causing touch of oiliness.

(Phases Configured by Components (j) to (n))

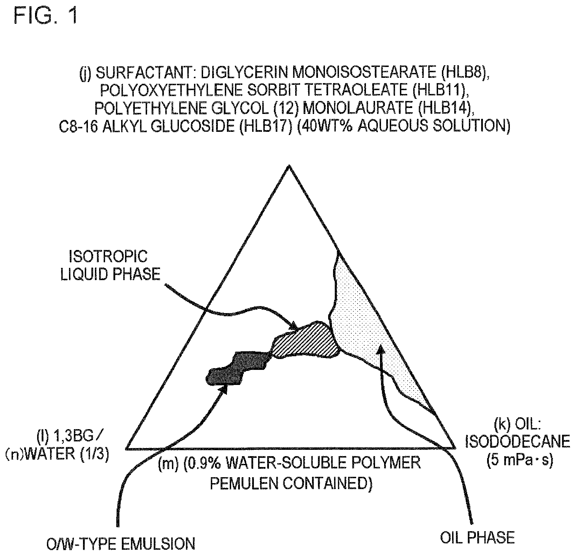

The cleansing agent composition used in the present invention is an O/W-type emulsion. The composition will be explained referring to a phase diagram of a quaternary system composed of the components (j), (k), (l) and (n) (see FIG. 1). In the phase diagram, three regions, that are an O/W-type emulsion area, and neighboring isotropic liquid phase area and oil phase area, appear as the water content decreases. Cleansing making use of these three phases takes advantages in an effect of dissolving hard solid impurities formed in the pores, in particular keratotic plugs, and an enhanced effect of removing keratotic plugs.

In the present invention, the initial state of the cleansing agent composition applied onto the skin is an O/W-type emulsion. By massaging the skin while keeping the form of O/W type in which the continuous phase consists of an aqueous phase, impurities around the pores and metabolites yielded from the skin may be swelled, and thereby the surficial impurities may readily be removed. Thereafter, the massage is continued depending on needs. During this process, water vaporizes off from the cleansing agent composition, and the continuous phase turns into an isotropic liquid phase consisting of the aqueous phase and the oil phase. Further massaging continued thereafter results in further vaporization of water, thereby the isotropic liquid phase turns into an oil phase, so that the massaging will continue while keeping the continuous phase as an oil phase.

Thereafter, upon rinsing of the skin with water by the user, the existing form of the cleansing agent composition varies sequentially from the oil phase, through the isotropic liquid phase, finally to the O/W-type emulsion, and may readily be removed from the skin without being adsorbed by the skin.

In the process of allowing three areas, that are the O/W-type emulsion, the isotropic liquid phase and the oil phase, to appear in the phase diagram of quaternary system composed of the components (j), (k), (l) and (n) as illustrated in the above, the ratio by weight (j)/(k) of the component (j) and the component (k) is adjusted to 3/7 to 3/1, so as to make appearances of the initial O/W-type emulsion area, and the isotropic liquid phase area after vaporization of water. In particular, by adjusting the ratio (j)/(k) to 1/1 to 7/3, the cosmetic may thoroughly be rinsed off without causing persistent feel of retention, while ensuring a sufficient level of cleansing power. The O/W-type emulsion may be formed by adjusting (j)/(k) to not more than 3/1, and the O/W-type emulsion may more readily be formed and may be changed through the isotropic liquid phase finally to the oil phase by adjusting not less than (j)/(k) to 3/7.

Moreover, by adjusting the ratio by weight (l)/(n) of the component (l) and the component (n) to 1/5 to 5/1, the cleansing agent composition will have the O/W-type emulsion area which is stable over a wide range, and the isotropic liquid phase area after vaporization of water. In particular, a sufficient level of cleansing power to be effected on keratotic plugs may be achieved by adjusting the ratio to 1/4 to 2/1, and further 1/2 to 2/1. The O/W-type emulsion may more readily be formed by adjusting (l)/(n) to not more than 5/1, and the O/W-type emulsion may more readily be changed through the isotropic liquid phase, finally to the oil phase, by adjusting (l)/(n) to not less than 1/5.

By virtue of the operations described in the above, the cleansing agent composition of this embodiment is now excellent in the effect of removing hard solid impurities formed in the pores, in particular keratotic plugs. The cleansing agent composition of this embodiment is used by applying it onto the skin and only needs rinsing with water thereafter, which is simple to use. The method is less stimulative, since the skin is cleansed with the agent. In addition, the cleansing agent composition of this embodiment is an O/W-type emulsion which has the continuous phase composed of an aqueous phase, so that the touch of skin after cleansing will be good.

Next, operations and effects of the cleansing agent composition according to the third embodiment of the present invention will be explained, referring to FIG. 1.

FIG. 1 is a phase diagram of a ternary system of the cleansing agent composition of the present invention, showing phase changes. FIG. 1 is a drawing illustrating an exemplary case where the 1,3-butylene glycol was used as the component (l), while adjusting the ratio by weight of water and the component (l) to 3:1. The component (m) used herein was 0.9% by weight of acrylate/ ($C_{10-30}$) alkyl methacrylate copolymer (PEMULEN TR-1, from Lubrizol Advanced Materials). The component (j) used herein was diglycerin monoisostearate (HLB8) (from Nisshin OilliO Group, Ltd. Cosmol 41V), polyoxyethylene sorbit tetraoleate (HLB11) (Rheodol 430, from KAO Corporation), polyethylene glycol (12) monolaurate (HLB14) (Emanone 1112HG, from KAO Corporation), and alkyl ($C_{8-16}$) glucoside (HLB17) (40%-by-weight aqueous solution) (MYDOL 10, from KAO Corporation), wherein the ratio by weight of them being adjusted to 10:6:22:15.

The composition is an O/W-type emulsion, turns to have the isotropic liquid phase (bicontinuous phase) during massage of the skin, and causes phase change to produce an oil phase in the process of further massage. More strongly solidified sebum inside the pores may be dissolved by the component (k), and thereby keratotic plugs clogging the pores may be removed. The composition illustrated in FIG. 1 exhibits a strong effect of removing keratotic plugs.

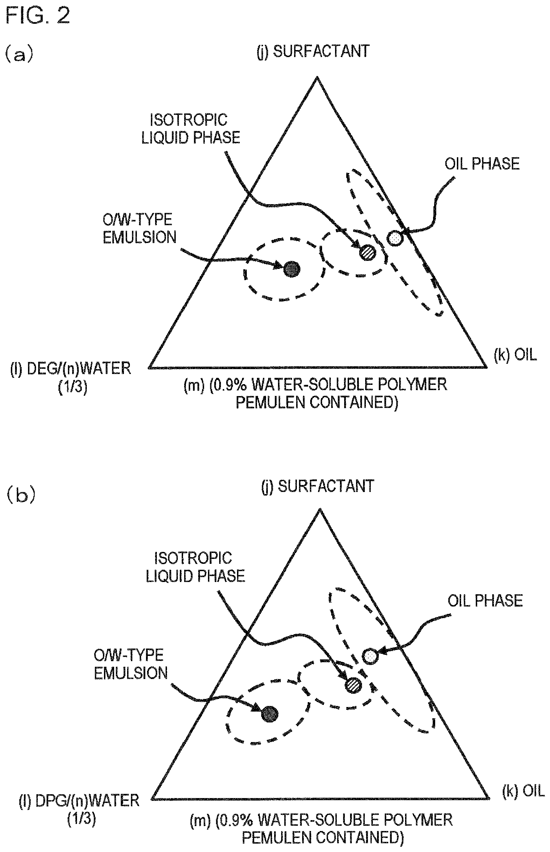

While the phase diagram illustrated in FIG. 1 relates to the case where 1,3-butylene glycol was used as the component (l), similar phase diagrams, as illustrated in FIG. 2, may be obtained and thereby similar operations and effects may be achieved, also by using other component (l) in place of 1,3-butylene glycol.

FIG. 2(a) is a phase diagram obtained when diethylene glycol was used as the component (l), and FIG. 2(b) is a phase diagram obtained when dipropylene glycol was used as the component (l). Ratios by weight of water and the component (l) was adjusted to 3:1, and content of the component (m) was adjusted to 0.9% by weight.

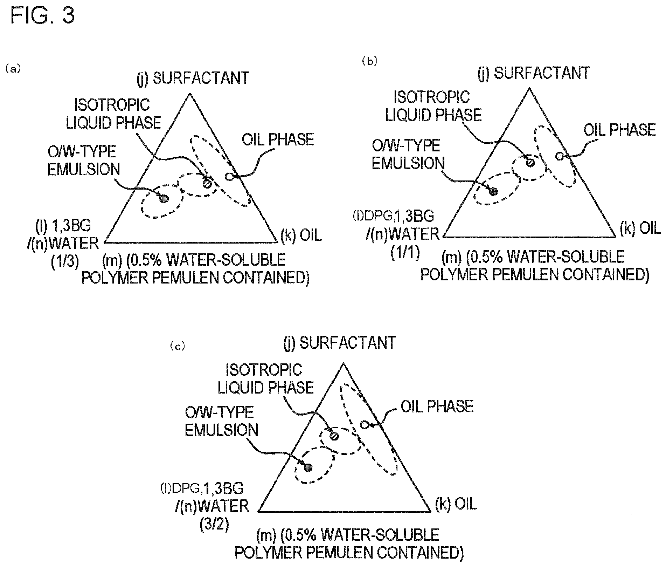

FIG. 3(a) to (c) are phase diagrams obtained under various ratios by weight of water and the component (l). The diagrams teaches that similar phase diagrams may be obtained also in these cases, and similar operations and effects may be obtained.

FIG. 3(a) is a phase diagram obtained when 1,3-butylene glycol was used as the component (l), while adjusting the ratio by weight of water and the component (l) to 3:1, and the content of the component (m) to 0.5% by weight.

FIG. 3(b) is a phase diagram obtained when dipropylene glycol and 1,3-butylene glycol were used as the component (l), while adjusting the ratio by weight of water and the component (l) to 1:1, and the content of the component (m) to 0.5% by weight.

Areas surrounded by broken lines in FIG. 3(b) represent phase changes shown by a composition of Example 21 described later.

FIG. 3(c) is a diagram obtained when dipropylene glycol and 1,3-butylene glycol were used as the component (l), while adjusting the ratio by weight of water and the component (l) to 2:3, and the content of the component (m) to 0.5% by weight.

Areas surrounded by broken lines in FIG. 3(c) represent phase changes shown by a composition of Example 22 described later.

The head portions of keratotic plugs exposed to the topmost surface of skin are configured by impurities around the pores and metabolites yielded from the skin entangled with each other. The impurities of this portion are more hard to be removed as compared with other types of impurities on the skin, and inhibit smooth removal of keratotic plugs. The bodies of keratotic plugs buried in the pores exist in the form of hard solid configured by sebum in the pores and metabolites yielded from the skin, and are hard to be removed since they obstruct the agent coming into the pores. The cleansing agent composition used in this embodiment causes phase changes in the process of cleansing from the O/W phase, through the isotropic liquid phase (bicontinuous phase), finally into the oil phase, and can thereby sequentially cleanse keratotic plugs making use of the phases respectively suitable for head portions and the bodies of keratotic plugs. Hard solid impurities in the pores, which could have been removed only by a sheet placed onto the skin, in particular keratotic plugs, may readily be removed simply by face washing. Further rinsing of the skin with water causes phase changes of the cleansing agent composition from the oil phase, through the isotropic liquid phase (bicontinuous phase), finally into the O/W phase. Since the final continuous phase is the aqueous phase, so that the refreshing touch of skin after cleansing may be obtained.

(Other Components)

The cleansing agent composition used in the present invention may further be added with components generally used for the cleansing agent, such as thickener, disinfectant, moisturizer, high-viscosity-oil, humectant, colorant, antiseptic, skin feel improver, perfume, antioxidant, and various liquid extracts, depending on needs.

The cleansing agent composition used in this embodiment preferably contains not so large amount of ionic surfactant from the viewpoint of building up a stable isotropic liquid phase, wherein the content is preferably less than 1.0%, more preferably not more than 0.1%, and particularly preferably zero.

The method of cleansing skin of the present invention may be used as face cleanser, makeup remover, body cleanser, massage agent, and keratotic plugs remover, particularly preferably used as face cleanser and keratotic plugs remover, and still more preferably used as keratotic plugs remover.

The method of cleansing skin of the present invention is applicable to skin having pores clogged with keratotic plugs. For example, areas having visible pores in the whole body, all over the face, and the area which extends across the forehead and down the nose (T-zone). Possible effects expected by removing keratotic plugs include improvement of visible pores, improvement of roughness, improvement of stickiness, and prevention of acne.

The cleansing agent composition used in the third embodiment may be obtained by homogenously mixing a compound having 2 to 6 carbon atoms and having one or two hydroxy group(s) (component (l)), a specific water-soluble polymer (component (m)), and water (component (n)), neutralizing the mixture with an alkali agent such as sodium hydroxide or potassium hydroxide, and then by adding a nonionic surfactant (component (j)) and an oil (component (k)). For the case where a material which stays in solid at room temperature is used, the cleansing agent composition may be manufactured by melting the material under heating, or by dissolving it into other component(s), and then by homogenously mixing the whole components.

[Examples]

Next, Examples of the present invention will be explained.

(Methods of Evaluation)

Effect of Removing Keratotic Plugs:

Five healthy panelists were recruited, and asked to cleanse their nostrils according to Examples and Comparative Examples described later. The numbers of keratotic plugs found on the nostril before and after the cleansing were counted.

Next, rate of removal of keratotic plugs was determined by (Equation 1), and evaluated.

Rate of removal of keratotic plugs = 100-(the number of keratotic plugs found in an 1-cm$^2$ area on the nostril after cleansing)/(the number of keratotic plugs found in an 1-cm$^2$ area on the nostril before cleansing)$\times$100 (Equation 1)

A: rate of removal of keratotic plugs $\geq$ 40%;
B: 40% > rate of removal of keratotic plugs $\geq$ 30%;
C: 30% > rate of removal of keratotic plugs $\geq$ 10%; and
D: 10% > rate of removal of keratotic plugs.

Cleansing agents listed in Tables 1 to 3 were respectively prepared (cleansing agents (A)-1 to (A)-5, (B)-1 to (B)-3, (C)-1 to (C)-6). Each of the cleansing agents (A)-1 to (A)-5 has the continuous phase consisting of an aqueous phase, and each of the cleansing agents (B)-1 to (B)-3 has the continuous phase consisting of an oil phase. Each of the cleansing agents (C)-1 to (C)-6 has the continuous phase consisting of an isotropic liquid phase (bicontinuous phase). States of these phases were confirmed by visual observation of appearance, observation under an optical polarizing microscope, drawing of phase diagram, and measurement of self-diffusion coefficient by NMR, and so forth.

As for the agents containing components which stay in liquid at room temperature, or those producing gel-like components as a result of mixing at room temperature, they were heated to 70 to 75°C under stirring for thorough dissolution, and then cooled to room temperature, to thereby obtain the cleansing agents.

(Example 1)

One gram of the cleansing agent (A)-1 was first applied only to the nose, followed by a 15-second massage. While leaving the agent (A)-1 adhered to the skin, one gram of the cleansing agent (B)-1 was then applied only to the nose, followed by a 15-second massage, further followed by a one-minute rinsing with water (see Table 4).

(Example 2)

One gram of the cleansing agent (A)-2 was first applied only to the nose, followed by a 15-second massage. While leaving the agent (A)-2 adhered to the skin, one gram of the cleansing agent (B)-2 was then applied only to the nose, followed by a 15-second massage, further followed by a one-minute rinsing with water (see Table 4).

(Example 3)

One gram of the cleansing agent (A)-3 was first applied only to the nose, followed by a 15-second massage. While leaving the agent (A)-3 adhered to the skin, one gram of the cleansing agent (B)-3 was then applied only to the nose, followed by a 15-second massage, further followed by a one-minute rinsing with water (see Table 4).

(Example 4)

One gram of the cleansing agent (A)-1 was first applied only to the nose, followed by a 10-second massage. While leaving the agent (A)-1 adhered to the skin, one gram of the cleansing agent (C)-1 was then applied only to the nose, followed by a 10-second massage. While leaving the agents (A)-1 and (C)-1 adhered to the skin, one gram of the cleansing agent (B)-1 was further applied only to the nose, followed by a 10-second massage, further followed by a one-minute rinsing with water (see Table 4).

(Example 5)

One gram of the cleansing agent (A)-1 was first applied only to the nose, followed by a 10-second massage. While leaving the agent (A)-1 adhered to the skin, one gram of the cleansing agent (C)-1 was then applied only to the nose, followed by a 10-second massage. While leaving the agents (A)-1 and (C)-1 adhered to the skin, one gram of the cleansing agent (B)-2 was further applied only to the nose, followed by a 10-second massage, further followed by a one-minute rinsing with water (see Table 4).

(Example 6)

One gram of the cleansing agent (A)-2 was first applied only to the nose, followed by a 10-second massage. While leaving the agent (A)-2 adhered to the skin, one gram of the cleansing agent (C)-2 was then applied only to the nose, followed by a 10-second massage. While leaving the agents (A)-2 and (C)-2 adhered to the skin, one gram of the cleansing agent (B)-2 was further applied only to the nose, followed by a 10-second massage, further followed by a one-minute rinsing with water (see Table 4).

(Example 7)

One gram of the cleansing agent (A)-3 was first applied only to the nose, followed by a 10-second massage. While leaving the agent (A)-3 adhered to the skin, one gram of the cleansing agent (C)-3 was then applied only to the nose, followed by a 10-second massage. While leaving the agents (A)-3 and (C)-3 adhered to the skin, one gram of the cleansing agent (B)-3 was further applied only to the nose, followed by a 10-second massage, further followed by a one-minute rinsing with water (see Table 4).

(Example 8)

One gram of the cleansing agent (A)-4 was first applied only to the nose, followed by a 10-second massage. While leaving the agent (A)-4 adhered to the skin, one gram of the cleansing agent (C)-3 was then applied only to the nose, followed by a 10-second massage. While leaving the agents (A)-4 and (C)-3 adhered to the skin, one gram of the cleansing agent (B)-3 was further applied only to the nose, followed by a 10-second massage, further followed by a one-minute rinsing with water (see Table 4).

(Example 9)

One gram of the cleansing agent (A)-4 was first applied only to the nose, followed by a 10-second massage. While leaving the agent (A)-4 adhered to the skin, one gram of the cleansing agent (C)-4 was then applied only to the nose, followed by a 10-second massage. While leaving the agents (A)-4 and (C)-4 adhered to the skin, one gram of the cleansing agent (B)-2 was further applied only to the nose, followed by a 10-second massage, further followed by a one-minute rinsing with water.

(Example 10)

One gram of the cleansing agent (A)-4 was first applied only to the nose, followed by a 10-second massage. While leaving the agent (A)-4 adhered to the skin, one gram of the cleansing agent (C)-5 was then applied only to the nose, followed by a 10-second massage. While leaving the agents (A)-4 and (C)-5 adhered to the skin, one gram of the cleansing agent (B)-2 was further applied only to the nose, followed by a 10-second massage, further followed by a one-minute rinsing with water.

(Example 11)

One gram of the cleansing agent (A)-5 was first applied only to the nose, followed by a 10-second massage. While leaving the agent (A)-5 adhered to the skin, one gram of the cleansing agent (C)-6 was then applied only to the nose, followed by a 10-second massage. While leaving the agents (A)-5 and (C)-6 adhered to the skin, one gram of the cleansing agent (B)-3 was further applied only to the nose, followed by a 10-second massage, further followed by a one-minute rinsing with water.

(Examples 12 to 31, and Example 32)

Cleansing agents listed in Table 5 and Table 6 (corresponds to Example 32) were prepared.
The components (l), (m) and (n) were optionally added with moisturizer(s) (sorbitol, glycerin), and optionally added with polypropylene glycol or hydrogenated polyisobutene, and each mixture was stirred to thereby homogeneously mix all components. The mixture was then neutralized by adding potassium hydroxide. The components (j), (k) and perfume were homogenously dispersed thereinto, to thereby obtain the cleansing agent compositions.
The cleansing agents were given a form of O/W-type emulsion. States of these phases were confirmed by visual observation of appearance, observation under an optical polarizing microscope, drawing of phase diagram, and measurement of self-diffusion coefficient by NMR, and so forth. Values of viscosity shown in Tables 2, 3, 5 and 6 are those measured by using a BM-type viscometer (from Tokimec Co., Ltd., measurement conditions: rotor No.1, 60 rpm, 30°C).
One gram of each of the thus-prepared cleansing agents was applied only to the nose, followed by a 30-second massage, further followed by a one-minute rinsing with water.
A phase change of the agent prepared in Example 21 was illustrated in FIG. 3(b), and a phase change of the agent prepared in Example 22 was illustrated in FIG. 3(c).
FIGs. 3(b) and (c) teach that, when the O/W-type agent having the continuous phase consisting of an aqueous phase is applied to the skin, water vaporizes off from the agent, and the agent is turned to have the isotropic liquid phase (bicontinuous phase). It is also understood that, when water further vaporizes off from the agent consisting of the isotropic liquid phase, the continuous phase changes into an oil phase, to thereby give the agent having the continuous phase consisting of the oil phase. Also with respect to other agents in Examples 12 to 32, when the O/W-type agent having the continuous phase consistingd of an aqueous phase is applied to the skin, water vaporizes off from the agent so as to turn the agent to have the isotropic liquid phase (bicontinuous phase), and when water further vaporizes off from the agent composed of the isotropic liquid phase, the continuous phase changes into an oil phase, to thereby give the agent having the continuous phase consisting of the oil phase.

(Comparative Example 1)

One gram of the cleansing agent (A)-3 was applied only to the nose, followed by a 30-second massage, further followed by a one-minute rinsing with water (see Table 7).

(Comparative Example 2)

One gram of the cleansing agent (A)-4 was applied only to the nose, followed by a 30-second massage, further followed by a one-minute rinsing with water (see Table 7).

(Comparative Example 3)

One gram of the cleansing agent (C)-2 was applied only to the nose, followed by a 30-second massage, further followed by a one-minute rinsing with water (see Table 7).

(Comparative Example 4)

One gram of the cleansing agent (C)-3 was applied only to the nose, followed by a 30-second massage, further followed by a one-minute rinsing with water (see Table 7).

(Comparative Example 5)

One gram of the cleansing agent (B)-2 was applied only to the nose, followed by a 30-second massage, further followed by a one-minute rinsing with water (see Table 7).

(Comparative Example 6)

One gram of the cleansing agent (B)-3 was applied only to the nose, followed by a 30-second massage, further followed by a one-minute rinsing with water (see Table 7).

(Comparative Example 7)

One gram of the cleansing agent (B)-1 was applied only to the nose, followed by a 15-second massage. While leaving the agent (B)-1 adhered to the skin, one gram of the cleansing agent (A)-1 was then applied only to the nose, followed by a 15-second massage, further followed by a one-minute rinsing with water (see Table 8).

Values of viscosity shown in Tables 2, 3, 5 and 6 are those measured by using a BM-type viscometer (from Tokimec Co., Ltd., measurement conditions: rotor No.1, 60 rpm, 30°C).

[Table 1]

| | | | Cleansing process (A) | | | | |
|---|---|---|---|---|---|---|---|
| | | | Cleansing agent (A) | | | | |
| | | Component (wt%) | (A)-1 | (A)-2 | (A)-3 | (A)-4 | (A)-5 |
| a | Poly(7)propylene glycol (ADEKA Carpol DL-30, from ADEKA Corporation | | | | 5.0 | 9.0 | 5.0 |
| | Dipropylene glycol | | | 15.0 | 8.0 | | 15.0 |
| | 1,3-Butylene glycol | | 50.0 | 15.0 | | 9.0 | |
| | Diethylene glycol monoethyl ether (Ethyl diglycol-NS, from Nippon Nyukazai Co., Ltd.) | | | | 8.0 | | 10.0 |
| b | Diglycerin monooleate (HLB:7)(Poem DO-100V, from Riken Vitamin Co., Ltd.) | | | | 7.0 | | 7.0 |
| | 2-Ethylhexyl glyceryl ether (HLB:7) (Penetol GE-EH, from KAO Corporation) | | | | 2.0 | | 2.0 |
| | Diglycerin monoisostearate (HLB:8) (Cosmol 41V, from Nisshin OilliO Group, Ltd.) | | | | | 7.0 | |
| | Polyoxyethylene coconut oil fatty acid glycerin (HLB:13) (Unigly MK-207G, from NOF Corporation) | | | | | 7.0 | |
| | Polyethylene glycol (PEG12) monolaurate (HLB:14) (Emanon 1112HG, from KAO Corporation) | | 5.0 | 25.0 | 14.0 | 8.0 | 19.0 |
| | Alkyl(C8-16) glucoside (HLB:17) (40wt% aqueous solution) (Mydol 10, from KAO Corporation) | | | | | 10.0 | 10.0 |
| c | Water | | 45.0 | 45.0 | 56.0 | 50.0 | 32.0 |
| | Total | | 100.0 | 100.0 | 100.0 | 100.0 | 100.0 |
| | Total HLB of component (b) | | 14.0 | 14.0 | 11.3 | 12.6 | 12.4 |

[Table 2]

| | | Component (wt%) | Cleansing process (B) | | |
|---|---|---|---|---|---|
| | | | Cleansing agent (B) | | |
| | | | (B)-1 | (B)-2 | (B)-3 |
| d | | Isododecane (5 mPa·s) | 20.0 | 20.0 | 15.0 |
| | | isopropyl myristate (10 mPa·s) | 10.0 | | |
| | | Hydrogenated polyisobutene (16.5 mPa·s) (ParLeam Ex, from NOF Corporation) | 20.0 | | 5.0 |
| | | Liquid paraffin (22.5 mPa·s) (Hicall K-230, from Kaneda Corporation) | 50.0 | 50.0 | 55.0 |
| Surfactant | | Diglycerin monoisostearate (HLB:8) (Cosmol 41V, from Nisshin OilliO Group, Ltd.) | | | 5.0 |
| | | Polyoxyethylene (20) sorbitan trioleate (HLB:11)(Rheodol TW-O320V, from KAO Corporation) | | 30.0 | |
| | | Polyoxyethylene coconut oil fatty acid glycerin (HLB:13) (Unigly MK-207G, from NOF Corporation) | | | 7.0 |
| | | Polyethylene glycol (PEG12) monolaurate (HLB:14) (Emanon 1112HG, from KAO Corporation) | | | 11.0 |
| | | Water | 0.0 | 0.0 | 0.1 |
| Moisturizer | | Glycerin | | | 1.9 |
| | | Total | 100.0 | 100.0 | 100.0 |

[Table 3]

| | | Component (wt%) | Cleansing process (C) | | | | | |
|---|---|---|---|---|---|---|---|---|
| | | | Cleansing agent (C) | | | | | |
| | | | (C)-1 | (C)-2 | (C)-3 | (C)-4 | (C)-5 | (C)-6 |
| e | | Isododecane (5 mPa·s) | 8.0 | 15.0 | 6.0 | 7.0 | 28.0 | 6.0 |
| | | Isopropyl myristate (10 mPa·s) | 8.0 | | 8.0 | | 28.0 | 6.0 |
| f | | Polyoxyethylene sorbit tetraoleate (HLB:11) (Rheodol 430, from KAO Corporation) | | | | 4.0 | | |
| | | Polyethylene glycol (PEG12) monolaurate (HLB: 14) (Emanon 1112HG, from KAO Corporation) | 14.0 | 14.0 | 14.0 | 20.0 | 12.8 | |
| | | Alkyl(C6-16) glucoside (HLB:17) (40wt% aqueous solution) (Mydol 10, from KAO Corporation) | 10.0 | 10.0 | 10.0 | | 3.0 | 20.0 |
| g | | Diglycerin monoisostearate (HLB:8) (Cosmol 41V, from Nisshin OilliO Group, Ltd.) | 7.0 | 7.0 | 7.0 | 2.0 | 10.0 | 1.5 |
| h | | 1,3-Butylene glycol | 15.0 | 15.0 | 20,0 | 50.0 | 10.0 | 12.0 |
| i | | Water | 38.0 | 24.0 | 18.0 | 15.0 | 8.2 | 53.0 |
| Others | | Hydrogenated polyisobutene (16.5 mPa·s) (ParLeam Ex, from NOF Corporation) | | | 2.0 | | | 1.5 |
| | | Sorbitol | | 15.0 | 15.0 | 2.0 | | |
| | | Total | 100.0 | 100.0 | 100.0 | 100.0 | 100.0 | 100.0 |

[Table 4]

| Cleansing process | Cleansing agent | Example 1 | Example 2 | Example 3 | Example 4 | Example 5 | Example 6 | Example 7 | Example 8 |
|---|---|---|---|---|---|---|---|---|---|
| Cleansing process (A) | Cleansing agent (A) | (A)-1 | (A)-2 | (A)-3 | (A)-1 | (A)-1 | (A)-2 | (A)-3 | (A)-4 |
| Cleansing process (C) | Cleansing agent (C) | | | | (C)-1 | (C)-1 | (C)-2 | (C)-3 | (C)-3 |
| Cleansing process (B) | Cleansing agent (B) | (B)-1 | (B)-2 | (H)-3 | (B)-1 | (B)-2 | (B)-2 | (B)-3 | (B)-3 |
| Effect of removing keratotic plugs | | B | B | B | A | A | A | A | A |

[Table 5]

| Component (wt%) | | Example | | | | | | | | | |
|---|---|---|---|---|---|---|---|---|---|---|---|
| | | 12 | 13 | 14 | 15 | 16 | 17 | 18 | 19 | 20 | 21 |
| j | Isostearyl glyceryl ether (HLB:5) (Penetol GE-IS, from KAO Corporation) | 5.0 | | | | | | | | | |
| | 2-Ethylhexyl glyceryl ether (HLB:7) (Penetol GE-EH, from KAO Corporation) | | | | | | | | | | |
| | Diglycerin monooleate (HLB:7)(Poem DO-100V, from Riken Vitamin Co., Ltd.) | | 7.0 | | | | | | | | |
| | Diglycerin monoisostearate (HLB:8) (Cosmol 41V, from Nisshin OilliO Group, Ltd.) | | | 5.0 | 7.0 | 7.0 | 7.0 | 7.0 | 7.0 | 7.0 | 7.0 |
| | Polyoxyethylene sorbit tetraoleate (HLB:11)(Rheodol 430, from KAO Corporation) | | | 3.0 | | | | | | | |
| | Polyoxyethylene coconut oil fatty acid glycerin (HLB:13)(Unigly MK-207G, from NOF Corporation) | | | | 7.0 | | | | | | |
| | Polyethylene glycol (PEG12) monolaurate (HLB:14)(Emanon 1112HG, from KAO Corporation) | 14.0 | 14.0 | 11.0 | 8.0 | 14.0 | 14.0 | 14.0 | 14.0 | 14.0 | 14.0 |
| | Alkyl(C8-16) glucoside (HLB:17) (40wt% aq.soln.)(Mydol 10, from KAO Corporation) | 10.0 | 10.0 | 7.5 | 10.0 | 10.0 | 10.0 | 10.0 | 10.0 | 10.0 | 10.0 |
| k | Isododecane (5 mPa·s) | 15.0 | 15.0 | 15.0 | 15.0 | 15.0 | 8.0 | 6.0 | 15.0 | 15.0 | 15.0 |
| | Isopropyl myristate (10 mPa·s) | | | | | | 8.0 | 8.0 | | | |
| l | Dipropylene glycol | 9.0 | 9.0 | 9.0 | 9.0 | 9.0 | 9.0 | 9.0 | 9.0 | | 12.0 |
| | 1,3-Butylene glycol | 9.0 | 9.0 | 9.0 | 9.0 | 9.0 | 9.0 | 9.0 | | 16.0 | 8.0 |
| | Diethylene glycol monoethyl ether (Ethyl diglycol-NS, from Nippon Nyukazai Co., Ltd.) | | | | | | | | 6.0 | | |
| m | Acrylates/C10-30 Alkyl Acrylate Crosspolymer (Carbopol ETD2020,from Lubrizol Advanced Materials, Inc.) | 0.5 | 0.5 | 0.5 | 0.5 | 0.5 | 0.5 | 0.5 | 0.5 | 0.5 | 0.5 |
| | (Acryloyldimethyl taurine ammonium/VP) copolymer (Aristoflex AVC, from Clariant) | | | | | | | | | | |
| | Water | Balance | Balance | Balance | Balance | Balance | Balance | Balance | Balance | Balance | Balance |
| Others | Hydrogenated polyisobutene (16.5 mPa·s) (ParLeam Ex, from NOF Corporation) | | | | | | | 2.0 | | | |
| | Sorbitol | | | | | | | | 10.0 | 5.0 | |
| | Glycerin | | | | 5.0 | | | | | | 7.0 |
| | Poly(7)propylene glycol (ADEKA Carpol DL-30, from ADEKA Corporation | | | | | | | | | | 5.0 |
| | Perfume | 0.2 | 0.2 | 0.2 | 0.2 | 0.2 | 0.2 | 0.2 | 0.2 | 0.2 | 0.2 |
| | 48% KOH | 0.3 | 0.3 | 0.3 | 0.3 | 0.3 | 0.3 | 0.3 | 0.3 | 0.3 | 0.3 |
| Total | | 100.0 | 100.0 | 100.0 | 100.0 | 100.0 | 100.0 | 100.0 | 100.0 | 100.0 | 100.0 |
| n | Total water | 43.156 | 41.1 | 44.1 | 35.1 | 41.1 | 40.1 | 40.1 | 34.1 | 38 | 27.1 |
| State of phase | | O/W-type emulsion | O/W-type emulsion | O/W-type emulsion | O/W-type emulsion | O/W-type emulsion | O/W-type emulsion | O/W-type emulsion | O/W-type emulsion | O/W-type emulsion | O/W-type emulsion |
| j/k | | 1.5 | 1.7 | 1.5 | 1.7 | 1.7 | 1.6 | 1.8 | 1.7 | 1.7 | 1.7 |
| Total HLB of component (j) | | 12.6 | 12.5 | 12.6 | 12.6 | 12.8 | 12.8 | 12.8 | 12.8 | 12.8 | 12.8 |
| l/n | | 0.42 | 0.44 | 0.41 | 0.51 | 0.44 | 0.45 | 0.45 | 0.44 | 0.42 | 0.74 |
| Rate of removal of keratotic plugs | | A | A | A | A | A | A | A | A | A | B |
| Touch of skin after cleansing | | 20 | 22 | 24 | 21 | 22 | 23 | 22 | 22 | 23 | 22 |

| Component (wt%) | | Example 22 | 23 | 24 | 25 | 26 | 27 | 28 | 29 | 30 | 31 |
|---|---|---|---|---|---|---|---|---|---|---|---|
| j | Isostearyl glyceryl ether (HLB:5) (Penetol GE-IS, from KAO Corporation) | | | | | | | | | 5.0 | |
| | 2-Ethylhexyl glyceryl ether (HLB:7) (Penetol GE-EH, from KAO Corporation) | 2.0 | | 0.5 | | | | | | 4.0 | |
| | Diglycerin monooleate (HLB:7)(Poem DO-100V, from Riken Vitamin Co., Ltd.) | | | | | | | | | | |
| | Diglycerin monoisostearate (HLB:8) (Cosmol 41V, from Nisshin OilliO Group, Ltd.) | 7.0 | 4.5 | 5.0 | 7.0 | 7.0 | 7.0 | 2.8 | 5.4 | 9.0 | 1.5 |
| | Polyoxyethylene sorbit tetraoleate (HLB:11) (Rheodol 430, from KAO Corporation) | | | | | | | | | | |
| | Polyoxyethylene coconut oil fatty acid glycerin (HLB:13) (Unigly MK-207G, from NOF Corporation) | | | | | | | | | | |
| | Polyethylene glycol (PEG12) monolaurate (HLB:14)(Emanon 1112HG, from KAO Corporation) | 14.0 | 9.0 | 10.0 | 14.0 | 14.0 | 14.0 | 5.6 | 10.8 | 12.0 | 11.0 |
| | Alkyl(C8-16) glucoside (HLB:17) (40wt% aq.soln.) (Mydol 10, from KAO Corporation) | 10.0 | 6.5 | 7.0 | 10.0 | 10.0 | 10.0 | 4.0 | 7.7 | 5.5 | 20.5 |
| k | Isododecane (5 mPa·s) | 15.0 | 12.0 | 4.0 | 15.0 | 15.0 | 15.0 | 11.5 | 10.0 | 15.0 | 15.0 |
| | Isopropyl myristate (10 mPa·s) | | 12.0 | 4.0 | | | | 11.5 | 10.0 | | |
| l | Dipropylene glycol | 9.0 | 20.0 | 8.0 | 9.0 | 9.0 | 10.0 | 20.0 | | 9.0 | 9.0 |
| | 1,3-Butylene glycol | 9.0 | | 8.0 | 9.0 | 6.0 | | 20.0 | 12.0 | 9.0 | 9.0 |
| | Diethylene glycol monoethyl ether (Ethyl diglycol-NS, from Nippon Nyukazai Co., Ltd.) | | | | | | | | | | |
| m | Acrylates/C10-30 Alkyl Acrylate Crosspolymer (Carbopol ETD2020,from Lubrizol Advanced Materials, Inc.) | 0.5 | 0.5 | 0.5 | 0.3 | 0.01 | 3.8 | 0.5 | 0.5 | 0.5 | 0.5 |
| | (Acryloyldimethyl taurine ammonium/VP) copolymer (Aristoflex AVC, from Clariant) | | | | 0.3 | | | | | | |
| | Water | Balance | Balance | Balance | Balance | Balance | Balance | Balance | Balance | Balance | Balance |
| Others | Hydrogenated polyisobutene (16.5 mPa·s) (ParLeam Ex, from NOF Corporation) | | | | | | | | | | |
| | Sorbitol | 15.0 | 15.0 | | 5.0 | 10.0 | | 5.7 | | | 15.0 |
| | Glycerin | | | 2.5 | | | | | | | 5.0 |
| | Poly(7)propylene glycol (ADEKA Carpol DL-30, from ADEKA Corporation) | 5.0 | | 2.5 | | | | | | | |
| | Perfume | 0.2 | 0.2 | 0.2 | 0.2 | 0.2 | 0.2 | 0.2 | 0.2 | 0.2 | 0.2 |
| | 48% KOH | 0.3 | 0.3 | 0.3 | 0.2 | 0.005 | 1.9 | 0.3 | 0.3 | 0.3 | 0.3 |
| Total | | 100.0 | 100.0 | 100.0 | 100.0 | 100.0 | 100.0 | 100.0 | 100.0 | 100.0 | 100.0 |
| n Total water | | 19.1 | 24.1 | 51.7 | 36.1 | 34.785 | 44.99 | 20.5 | 47.8 | 33.9 | 25.5 |
| State of phase | | O/W-type emulsion | O/W-type emulsion | O/W-type emulsion | O/W-type emulsion | O/W-type emulsion | O/W-type emulsion | O/W-type emulsion | O/W-type emulsion | O/W-type emulsion | O/W-type emulsion |
| j/k | | 1.8 | 0.7 | 2.3 | 1.7 | 1.7 | 1.7 | 0.434782609 | 1.0 | 2.1 | 1.4 |
| Total HLB of component (j) | | 12.4 | 12.8 | 12.6 | 12.8 | 12.8 | 12.8 | 12.8 | 12.8 | 10.3 | 14.8 |
| l/n | | 0.94 | 0.83 | 0.31 | 0.5 | 0.43 | 0.22 | 1.95 | 0.25 | 0.53 | 0.71 |
| Rate of removal of keratotic plugs | | B | B | B | A | B | B | A | B | A | B |
| Touch of skin after cleansing | | 21 | 21 | 22 | 22 | 23 | 21 | 20 | 22 | 23 | 22 |

[Table 6]

| Component | | | (wt%) |
|---|---|---|---|
| j | | Diglycerin monoisostearate (HLB:8) (Cosmol 41V, from Nisshin OilliO Group, Ltd.) | 7.00 |
| | | Polyethylene glycol (PEG12) monolaurate (HLB:14)(Emanon 1112HG, from KAO Corporation) | 14.00 |
| | | Alkyl(C8-16) glucoside (HLB:17) (40wt% aqueous solution) (Mydol 10, from KAO Corporation) | 10.00 |
| k | | Isododecane (5 mPa·s) | 15.00 |

(continued)

|   | | Component | (wt%) |
|---|---|---|---|
| l | | Dipropylene glycol | 9.00 |
| | | 1,3-Butylene glycol | 9.00 |
| m | | (C14-16)Alkyl acrylate/steareth-20 methacrylate copolymer(Aculyn 22, from Rohm and Haas Company) | 0.50 |
| n | | Water | 35.05 |
| Others | | Perfume | 0.20 |
| | | 48% KOH | 0.25 |
| | | Total | 100.00 |
| | | State of phase | O/W-type emulsion |
| | | j/k | 1.7 |
| | | Total HLB of component (j) | 12.8 |
| | | l/n | 0.5 |
| | | Effect of removing keratotic plugs | A |

[Table 7]

| Cleansing process | Cleansing agent | Comparative Example 1 | Comparative Example 2 | Comparative Example 3 | Comparative Example 4 | Comparative Example 5 | Comparative Example 6 |
|---|---|---|---|---|---|---|---|
| Cleansing process (A) | Cleansing agent (A) | (A)-3 | (A)-4 | | | | |
| Cleansing process (C) | Cleansing agent (C) | | | (C)-2 | (C)-3 | | |
| Cleansing process (B) | Cleansing agent (B) | | | | | (B)-2 | (B)-3 |
| Effect of removing keratotic plugs | | D | D | C | C | D | D |

[Table 8]

| Cleansing process | Cleansing agent | Comparative Example 7 |
|---|---|---|
| Cleansing process (B) | Cleansing agent (B) | (B)-1 |
| Cleansing process (C) | Cleansing agent (C) | |
| Cleansing process (A) | Cleansing agent (A) | (A)-1 |
| Effect of removing keratotic plugs | | D |

From Table 4, Examples 1 to 8 were found to show excellent effects of removing keratotic plugs. Excellent effects of removing keratotic plugs were obtained also in Examples 9 to 11.

From Tables 5 and 6, also Example 12 to 32 were found to show excellent effects of removing keratotic plugs.

The method of cleansing in all Examples is based on application of agent, massage and rinsing, and is found to be less stimulative. The method adopted in Examples 1 to 32 is laborsaving since there is no need of drying pack or the like.

On the other hand, from Tables 7 and 8, Comparative Examples 1 to 7 were found to show poor effects of removing keratotic plugs.

The cleansing agent compositions prepared in Examples 12 to 31 were evaluated also with respect to the touch of skin after cleansing as described below.

By a participation of eight female panelists, the touch of skin after cleansing were evaluated by sensory test, and total scores of eight persons were determined. Results are shown in Table 5.

3: the skin was felt rich and moistened;
2: the skin was felt slightly sticky or slightly desiccated; and
1: the skin was felt sticky or desiccated.

The cleansing agent composition prepared in Examples 12 to 31 were found to give good touch of skin after cleansing.

**Claims**

1. A method of cleansing skin comprising:

   (A) massaging the skin using an agent in the form of O/W-type emulsion, which contains an oil, a compound having 2-6 carbon atoms and having one or two hydroxyl group(s), a surfactant and water, and has a continuous phase consisting of an aqueous phase;
   (C) massaging the skin using an agent consisting of an isotropic liquid phase, obtained by allowing water to vaporize off from the agent, so as to turn each of the oil phase and the aqueous phase into a continuous phase to thereby form the isotropic liquid phase; and
   (B) massaging the skin using an agent which has a continuous phase consisting of an oil phase, obtained by allowing water to vaporize off from the agent consisting of the isotropic liquid phase, so as to turn the continuous phase into the oil phase.

2. A method of cleansing skin comprising:

   (A) massaging the skin using an agent which contains a water-miscible solvent, a surfactant and water, the content of the water-miscible solvent being 10% by weight or more, and has a continuous phase consisting of an aqueous phase; and
   (B) massaging the skin using an agent which has a continuous phase consisting of an oil phase,

   wherein the process (A) precedes, and the process (B) succeeds immediately thereafter while leaving the agent of process (A) unremoved on the skin.

3. A method of cleansing skin comprising:

(A) massaging the skin using an agent which contains a water-miscible solvent, a surfactant and water, and has a continuous phase consisting of an aqueous phase;

(C) massaging the skin using an agent which contains an oil, a surfactant, a water-miscible solvent and water, and consists of an isotropic liquid phase in which each of an oil phase and an aqueous phase configures a continuous phase; and

(B) massaging the skin using an agent which has a continuous phase consisting of an oil phase,

wherein the process (A) precedes, the process (C) comes next, and the process (B) succeeds.

4. The method of cleansing skin according to Claim 1,
wherein the agent in the form of O/W-type emulsion contains components (j) to (n) below:

(j) not less than 5% by weight, and not more than 50% by weight of a nonionic surfactant which is configured by a single species of nonionic surfactant, or a mixed surfactant having two or more species of nonionic surfactants mixed therein, and has an HLB value of the single species of nonionic surfactant or the mixed surfactant of not less than 10, and not more than 15;
(k) not less than 10% by weight, and not more than 39% by weight of an oil having a viscosity at 30°C of not more than 15 mPa·s;
(l) not less than 10% by weight, and not more than 50% by weight of a compound which has 2 to 6 carbon atoms and one or two hydroxy group(s);
(m) not less than 0.01% by weight, and not more than 5% by weight of a polymer selected from water-soluble polymer which contains (meth)acrylic acid as a constituent, and acryloylmethyl taurate/vinyl pyrrolidone copolymer; and
(n) not less than 10% by weight, and not more than 50% by weight of water,

wherein a ratio by weight given by (j)/(k) is not less than 3/7, and not more than 3/1.

5. The method of cleansing skin according to Claim 4,
wherein a ratio by weight given by (l)/(n) is not less than 1/5, and not more than 5/1.

6. The method of cleansing skin according to Claim 4 or 5,
wherein the (m) water-soluble polymer is an acrylate/ alkyl methacrylate copolymer.

7. The method of cleansing skin according to any one of Claims 4 to 6,
wherein the component (l) is a glycol.

8. The method of cleansing skin according to any one of Claims 1 to 7, further comprising:
(D) adding water so as to remove the agent from the skin, succeeding to the massaging of skin in the process (B).

9. The method of cleansing skin according to any one of Claims 1 to 8, configured to remove keratotic plugs in the skin.

**Patentansprüche**

1. Verfahren zum Reinigen der Haut, umfassend

(A) Massieren der Haut unter Verwendung eines Mittels in Form einer Emulsion des Typs Öl/Wasser, die ein Öl, eine Verbindung mit 2-6 Kohlenstoffatomen und einer oder zwei Hydroxylgruppe(n), ein Tensid und Wasser enthält, und die eine kontinuierliche Phase, welche aus einer wässrigen Phase besteht, aufweist;
(C) Massieren der Haut unter Verwendung eines Mittels, welches aus einer isotropen flüssigen Phase besteht, erhalten durch Verdampfen von Wasser aus dem Mittel, so dass sowohl die Ölphase als auch die wässrige Phase in eine kontinuierliche Phase umgewandelt werden, um dadurch die isotrope flüssige Phase zu bilden; und
(B) Massieren der Haut unter Verwendung eines Mittels, welches eine kontinuierliche Phase aufweist, die aus einer Ölphase besteht, erhalten durch Verdampfen von Wasser aus dem Mittel, das aus der isotropen flüssigen Phase besteht, so dass die kontinuierliche Phase in die Ölphase übergeht.

2. Verfahren zum Reinigen der Haut, umfassend

(A) Massieren der Haut unter Verwendung eines Mittels, welches ein mit Wasser mischbares Lösungsmittel, ein Tensid und Wasser enthält, wobei der Gehalt des mit Wasser mischbaren Lösungsmittels 10 Gew.-% oder mehr beträgt, und welches eine kontinuierliche Phase, die aus einer wässrigen Phase besteht, aufweist; und
(B) Massieren der Haut unter Verwendung eines Mittels, welches eine kontinuierliche Phase, die aus einer Ölphase besteht, aufweist,

wobei der Vorgang (A) vorangeht und der Vorgang (B) unmittelbar danach folgt, während das Mittel des Vorgangs (A) auf der Haut nicht entfernt wird.

3. Verfahren zum Reinigen der Haut, umfassend

(A) Massieren der Haut unter Verwendung eines Mittels, welches ein mit Wasser mischbares Lösungsmittel, ein Tensid und Wasser enthält, und welches eine kontinuierliche Phase, die aus einer wässrigen Phase besteht, aufweist;
(C) Massieren der Haut unter Verwendung eines Mittels, welches ein Öl, ein Tensid, ein mit Wasser mischbares Lösungsmittel und Wasser enthält, und welches aus einer isotropen flüssigen Phase besteht, in der sowohl eine Ölphase als auch eine wässrige Phase eine kontinuierliche Phase bilden; und
(B) Massieren der Haut unter Verwendung eines Mittels, welches eine kontinuierliche Phase, die aus einer Ölphase besteht, aufweist,

wobei der Vorgang (A) vorangeht, der Vorgang (C) als nächstes folgt und der Vorgang (B) danach folgt.

4. Verfahren zum Reinigen der Haut gemäß Anspruch 1,
wobei das Mittel in Form einer Emulsion des Typs Öl/Wasser die folgenden Komponenten (j) bis (n) enthält

(j) nicht weniger als 5 Gew.-% und nicht mehr als 50 Gew.-% eines nichtionischen Tensids, welches durch eine einzelne Spezies eines nichtionischen Tensids oder durch ein gemischtes Tensid mit zwei oder mehr darin gemischten Spezies von nichtionischen Tensiden gebildet ist, und welches einen HLB-Wert der einzelnen Spezies des nichtionischen Tensids oder des gemischten Tensids von nicht weniger als 10 und nicht mehr als 15 aufweist;
(k) nicht weniger als 10 Gew.-% und nicht mehr als 39 Gew.-% eines Öls mit einer Viskosität bei 30°C von nicht mehr als 15 mPa·s;
(l) nicht weniger als 10 Gew.-% und nicht mehr als 50 Gew.-% einer Verbindung mit 2 bis 6 Kohlenstoffatomen und einer oder zwei Hydroxygruppe(n);
(m) nicht weniger als 0,01 Gew.-% und nicht mehr als 5 Gew.-% eines Polymers, ausgewählt aus einem wasserlöslichen Polymer, das (Meth)acrylsäure als Bestandteil enthält, und einem Acryloylmethyltaurat/Vinylpyrrolidon-Copolymer; und
n) nicht weniger als 10 Gew.-% und nicht mehr als 50 Gew.-% Wasser,

wobei ein durch (j)/(k) gegebenes Gewichtsverhältnis nicht weniger als 3/7 und nicht mehr als 3/1 beträgt.

5. Verfahren zum Reinigen der Haut gemäß Anspruch 4,
wobei ein durch (l)/(n) gegebenes Gewichtsverhältnis nicht weniger als 1/5 und nicht mehr als 5/1 beträgt.

6. Verfahren zum Reinigen der Haut gemäß Anspruch 4 oder 5,
wobei das wasserlösliche Polymer (m) ein Acrylat/Alkylmethacrylat-Copolymer ist.

7. Verfahren zum Reinigen der Haut gemäß mindestens einem der Ansprüche 4 bis 6,
wobei die Komponente (l) ein Glykol ist.

8. Verfahren zum Reinigen der Haut gemäß mindestens einem der Ansprüche 1 bis 7, das weiterhin umfasst
(D) Zugabe von Wasser, um das Mittel von der Haut zu entfernen, im Anschluss an das Massieren der Haut in dem Vorgang (B).

9. Verfahren zum Reinigen der Haut gemäß mindestens einem der Ansprüche 1 bis 8, das zum Entfernen keratöser Komedone in der Haut ausgestaltet ist.

**Revendications**

1. Procédé de nettoyage de la peau comprenant :

   (A) le massage de la peau en utilisant un agent sous la forme d'une émulsion de type O/W, qui contient une huile, un composé ayant 2-6 atomes de carbone et ayant un ou deux groupements hydroxyle, un agent tensioactif et de l'eau et a une phase continue constituée d'une phase aqueuse ;
   (C) le massage de la peau en utilisant un agent constitué d'une phase de liquide isotrope obtenue en permettant à l'eau de se vaporiser de l'agent de manière à convertir chacune de la phase huileuse et de la phase aqueuse en une phase continue en sorte de former la phase de liquide isotrope ; et
   (B) le massage de la peau en utilisant un agent qui a une phase continue constituée d'une phase huileuse obtenue en permettant à l'eau de se vaporiser de l'agent constitué de la phase de liquide isotrope de manière à convertir la phase continue en phase huileuse.

2. Procédé de nettoyage de la peau comprenant :

   (A) le massage de la peau en utilisant un agent qui contient un solvant miscible à l'eau, un agent tensioactif et de l'eau, le contenu du solvant miscible à l'eau étant de 10 % en poids ou plus, et a une phase continue constituée d'une phase aqueuse ; et
   (B) le massage de la peau en utilisant un agent qui a une phase continue constituée d'une phase huileuse,

   dans lequel le procédé (A) précède et le procédé (B) suit immédiatement après en laissant l'agent du procédé (A) non retiré de la peau.

3. Procédé de nettoyage de la peau comprenant :

   (A) le massage de la peau en utilisant un agent qui contient un solvant miscible à l'eau, un agent tensioactif et de l'eau et a une phase continue constituée d'une phase aqueuse ;
   (C) le massage de la peau en utilisant un agent qui contient une huile, un agent tensioactif, un solvant miscible à l'eau et de l'eau et est constitué d'une phase de liquide isotrope dans laquelle chacune d'une phase huileuse et d'une phase aqueuse configure une phase continue ;
   (B) un massage de la peau en utilisant un agent qui a une phase continue constituée d'une phase huileuse,

   dans lequel le procédé (A) précède, le procédé (C) vient ensuite et le procédé (B) suit.

4. Procédé de nettoyage de la peau selon la revendication 1,
   dans lequel l'agent sous la forme d'une émulsion de type O/W contient les composants (j) à (n) ci-dessous :

   (j) pas moins de 5 % en poids et pas plus de 50 % en poids d'un agent tensioactif non ionique qui est configuré par une seule espèce d'agent tensioactif non ionique, ou un agent tensioactif mixte ayant deux ou plus d'espèces d'agents tensioactifs non ioniques qui y sont mélangés, et a une valeur HLB de la seule espèce d'agent tensioactif non ionique ou de l'agent tensioactif mixte de pas moins de 10 et de pas plus de 15 ;
   (k) pas moins de 10 % en poids et pas plus de 39 % en poids d'une huile ayant une viscosité à 30 °C de pas plus de 15 mPa.s ;
   (l) pas moins de 10 % en poids et pas plus de 50 % en poids d'un composé qui a 2 à 6 atomes de carbone et un ou deux groupements hydroxy ;
   (m) pas moins de 0,01 % en poids et pas plus de 5 % en poids d'un polymère choisi parmi un polymère hydrosoluble qui contient de l'acide (méth)acrylique comme constituant et d'un copolymère de taurate d'acryloylméthyle et de pyrrolidone de vinyle ; et
   (n) par moins de 10 % en poids et pas plus de 50 % en poids d'eau,

   dans lequel un rapport en poids donné par (j)/(k) n'est pas inférieur à 3/7 et pas supérieur à 3/1.

5. Procédé de nettoyage de la peau selon la revendication 4,
   dans lequel un rapport en poids donné par (1)/(n) n'est pas inférieur à 1/5 et pas supérieur à 5/1.

6. Procédé de nettoyage de la peau selon la revendication 4 ou 5,
   dans lequel le polymère hydrosoluble (m) est un copolymère d'acrylate et de méthacrylate d'alkyle.

**7.** Procédé de nettoyage de la peau selon l'une quelconque des revendications 4 à 6,
dans lequel le composant (I) est un glycol.

**8.** Procédé de nettoyage de la peau selon l'une quelconque des revendications 1 à 7, comprenant en outre :
(D) l'addition d'eau de manière à éliminer l'agent de la peau pour passer au massage de la peau dans le procédé (B).

**9.** Procédé de nettoyage de la peau selon l'une quelconque des revendications 1 à 8, configuré pour éliminer des
bouchons kératosiques dans la peau.

# FIG. 1

(j) SURFACTANT: DIGLYCERIN MONOISOSTEARATE (HLB8),
POLYOXYETHYLENE SORBIT TETRAOLEATE (HLB11),
POLYETHYLENE GLYCOL (12) MONOLAURATE (HLB14),
C8-16 ALKYL GLUCOSIDE (HLB17) (40WT% AQUEOUS SOLUTION)

ISOTROPIC
LIQUID PHASE

(k) OIL:
ISODODECANE
(5 mPa·s)

(l) 1,3BG /
(n)WATER (1/3)

(m) (0.9% WATER-SOLUBLE POLYMER
PEMULEN CONTAINED)

O/W-TYPE EMULSION

OIL PHASE

FIG. 2

(a)

(j) SURFACTANT

ISOTROPIC
LIQUID PHASE

OIL PHASE

O/W-TYPE
EMULSION

(l) DEG/(n)WATER
(1/3)

(k) OIL

(m) (0.9% WATER-SOLUBLE POLYMER
PEMULEN CONTAINED)

(b)

(j) SURFACTANT

ISOTROPIC
LIQUID PHASE

OIL PHASE

O/W-TYPE
EMULSION

(l) DPG/(n)WATER
(1/3)

(k) OIL

(m) (0.9% WATER-SOLUBLE POLYMER
PEMULEN CONTAINED)

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007025947 A
- EP 1433476 A
- WO 9912640 A
- US 5034228 A
- JP 2009013092 A
- US 3277013 A

- JP H09194325 A
- JP H08109119 A
- JP 2002241260 A
- JP 2004217640 A
- JP 2008184413 A
- JP 2008184414 A

**Non-patent literature cited in the description**

- **SAGITANI et al.** *Yukagaku,* 1984, vol. 33 (3), 156-161